# EUROPEAN PATENT APPLICATION

(11) **EP 4 675 629 A1**
(43) Date of publication of application: **07.01.2026**
(21) Application number: 24186167.3
(22) Date of filing: 03.07.2024
(51) Int. Cl.: G16B 15/30, G16B 40/20, G16B 35/00

(54) **COMPUTER-IMPLEMENTED METHOD AND SYSTEM FOR RANKING AND/OR IDENTIFYING SUBSTRATE-ENZYME COMBINATIONS AND PREDICTING SUBSTRATE RANGE USING MACHINE LEARNING**

(71) Applicant: Hedera-22 SA, 4000 Liège (BE)
(72) Inventor: LA ROCCA, Raphaël, 4000 Liège (BE); NAÔMÉ, Aymeric, 4000 Liège (BE); GOFFIN, Philippe, 4000 Liège (BE); REZENDE, Lucas C. D., 4000 Liège (BE)
(74) Representative: Brantsandpatents bv

(57) **Abstract**

The current invention relates to a computer-implemented method for ranking and/or identifying substrate-enzyme combinations according to the likelihood that a substrate undergoes catalytic conversion by an enzyme or inhibits the enzyme; to a computer system; and a computer program product. The invention also relates to uses of the method for predicting the substrate range of an enzyme X, for screening enzyme variants with altered substrate specificity and/or catalytic activity in relation to a substrate of interest, and for predicting biological phenotypes associated with an enzyme X.

## Description

### FIELD OF THE INVENTION

The present invention pertains to the use of computer-implemented methods and systems for ranking and/or identifying substrate-enzyme combinations based on their likelihood of catalytic conversion. The invention also includes the application of machine learning models for predicting the substrate range of an enzyme, for screening enzyme variants with altered substrate specificity and/or catalytic activity, and for the prediction of a biological phenotype associated with an enzyme.

### BACKGROUND

The field of molecular docking has long been instrumental in elucidating enzyme-substrate interactions, offering insights into substrate identification, enzyme engineering, and the discovery of potent enzyme inhibitors.

The conventional molecular docking paradigm in enzyme-substrate interaction studies revolves around the binding of ligands (substrates or inhibitors) to a singular 3D model of a protein or ribozyme (receptor, enzyme), with their binding affinity assessed through a scoring system. This method, however, neglects the dynamic nature of enzymes in solution, where various conformations coexist. Additionally, the transition from an enzyme's apo (unbound) state to a ligand-bound complex often involves conformational changes. Efforts to introduce flexibility by considering selected side-chain residues have been made, but the persistent rigidity of the enzyme backbone remains a limitation. The conventional approach lacks consideration for the dynamic equilibrium of enzyme conformations.

The catalytic role of enzymes, aimed at modifying the rate of reactions, introduces another challenge. Enzymes exhibit a higher affinity for reaction intermediates compared to substrates/products. Previous approaches have utilized reaction intermediates for substrate prediction, yet this concept is not widely adopted in a refined manner. Furthermore, the conventional docking methodology typically ranks ligands based solely on binding affinity scores, disregarding the necessity for ligands to undergo a reaction for enzymatic transformation. This limitation results in an incomplete understanding of substrate selection.

In summary, the conventional molecular docking approach is hindered by the lack of consideration for the dynamic nature of enzymes in solution, the limited focus on reaction intermediates, and the exclusive reliance on binding affinity scores for substrate selection. These challenges highlight the need for innovative methodologies to address the shortcomings of traditional approaches in enzyme-substrate interaction studies.

### SUMMARY OF THE INVENTION

The present invention and embodiments thereof serve to provide a solution to one or more of above-mentioned disadvantages. To this end, the present invention relates to a computer-implemented method for ranking and/or identifying substrate-enzyme combinations according to the likelihood that a substrate undergoes catalytic conversion by an enzyme or inhibits the enzyme according to claim 1.

In particular, the method comprises the following steps:
a) obtaining a 3D structure for each j conformations of one or more enzymes of a same family,
b) obtaining a 3D representation of reaction intermediates of each of i possible substrates of said j conformations in every possible stereochemical configuration z of the reaction intermediate,
c) molecular docking said 3D representations of the reaction intermediates of step b) against each 3D structure of said j conformations of step a), thereby generating a plurality of s docking poses of the substrate in the active site for each izj combination;
d) for each izjs docking pose obtained in step c), deriving a vector of information that represents the docking pose for each of the enzyme with the substrate, the enzyme with the reaction intermediate, and the enzyme with the product of the substrate;
e) optionally where in step d) the vector of information does not comprise interaction energies, converting the vector of information to interaction energies for each of the enzyme with the substrate, the enzyme with the reaction intermediate, and the enzyme with the product of the substrate;
f) training a machine learning model:
   - using a one-step approach, on a training dataset comprising a subset of the i possible substrates and a first subset of the one or more enzymes and the associated likelihoods of each substrate of the subset of substrates undergoing catalytic conversion by the enzymes of the first subset or inhibiting the enzymes of the first subset based on vectors of information as in step d) or interaction energies as in step e), thereby obtaining a machine learning model trained to provide a ranking and/or to identify substrate-enzyme combinations according to the likelihood that a substrate undergoes catalytic conversion by an enzyme or inhibits the enzyme based on the vector of information or interaction energies of each of the enzyme with the substrate, the enzyme with the reaction intermediate, and the enzyme with the product of the substrate,
      or
   - using a two-step approach comprising a first and a second step, the first step comprising training the machine learning model on a training dataset comprising vectors of information as in step d) or interaction energies as in step e), and their associated likelihoods of each substrate undergoing catalytic conversion by the enzymes or inhibiting the enzymes based on their determined interaction energies or vector of information, and the second step comprising training the machine learning model on a training dataset comprising likelihoods of each substrate undergoing catalytic conversion by the enzymes or inhibiting the enzymes based on their vectors of information or determined interaction energies, and their associated likelihoods of the enzymes providing a certain biological phenotype,
      thereby obtaining a machine learning model trained: to provide a ranking and/or to identify substrate-enzyme combinations according to the likelihood that a substrate undergoes catalytic conversion by an enzyme or inhibits the enzyme based on vectors of information or interaction energies of each of the enzyme with the substrate, the enzyme with the reaction intermediate, and the enzyme with the product of the substrate, and
      to provide a likelihood of an enzyme providing a certain biological phenotype according to the likelihood that a substrate undergoes catalytic conversion by an enzyme or inhibits the enzyme based on the vector of information or on interaction energies of each of the enzyme with the substrate, the enzyme with the reaction intermediate, and the enzyme with the product of the substrate; and
g) based on vectors of information and/or interaction energies determined in step d) or e), determining the likelihood that the substrate undergoes catalytic conversion by the enzyme or inhibits the enzyme, using the trained model of step f), and subsequently providing a ranking and/or identifying substrate-enzyme combinations based on said likelihood.

Preferred embodiments of the computer-implemented method are shown in any of the claims 2 to 7.

In a second and third aspect, the present invention relates to a computer system and computer program product according to claims 8 and 9 respectively, both for ranking and/or identifying substrate-enzyme combinations according to the likelihood that a substrate undergoes catalytic conversion by an enzyme.

In other aspects, the invention relates to uses of the computer-implemented method for predicting the substrate range of an enzyme X according to claim 10, for screening enzyme variants with altered substrate specificity and/or catalytic activity in relation to a substrate of interest according to claim 12, and for predicting the biological phenotype associated with an enzyme X according to claim 18.
in other aspects, the invention also relates to a computer-implemented method of predicting the substrate range of an enzyme X according to claim 11, to a computer-implemented method of screening enzyme variants with altered substrate specificity and/or catalytic activity in relation to a substrate of interest according to claim 13, and to a computer-implemented method of predicting the biological phenotype associated with an enzyme X according to claim 19, each of the methods in particular using the computer implemented method of any one of claims 1-7.

The proposed method for ranking and/or identifying substrate-enzyme combinations offers enhanced accuracy by employing multiple 3D enzyme structures and considering both binding strength and reaction speed. Utilizing molecular docking and machine learning, the method provides comparing all substrates for any given enzyme in terms of their overall rate of transformation, which is indicative of efficient substrate conversion. This approach enables the prediction of enzyme substrate ranges, screening of enzyme variants with altered specificity and/or catalytic activity, and predicting of biological phenotypes associated with a particular enzyme.

### DESCRIPTION OF FIGURES

The following description of the figures of specific embodiments of the invention is merely exemplary in nature and is not intended to limit the present teachings, their application or uses.

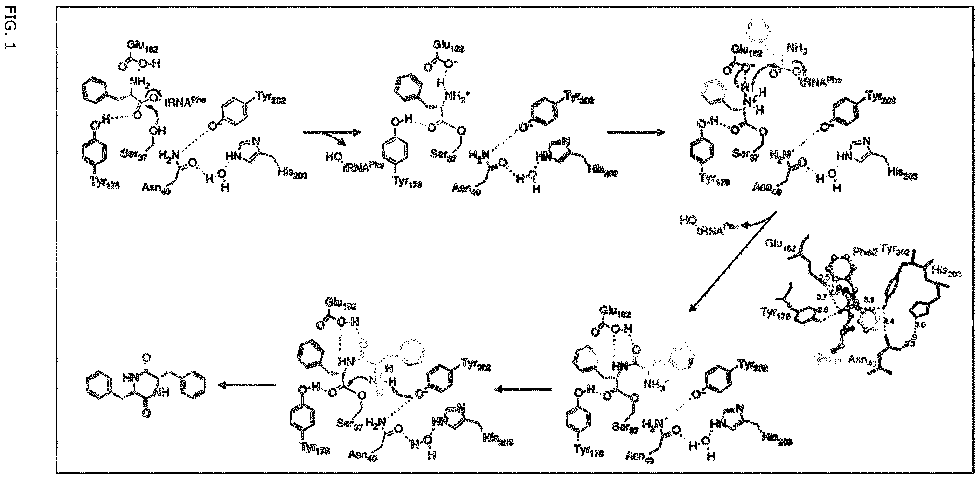

**Figure 1** (FIG. 1) shows a representation of the catalytic cycle of the cyclodipeptide synthase (CDPS) AlbC, which catalyzes the synthesis of a di-phenylalanine cyclic dipeptide.

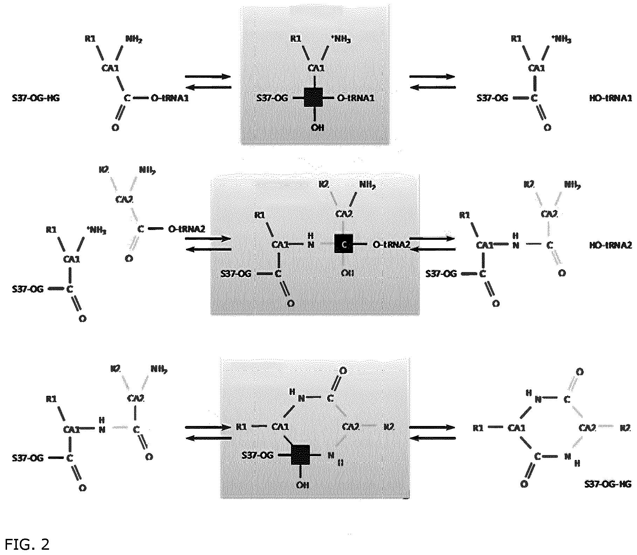

**Figure 2** (FIG. 2) shows the breakdown of the reaction catalyzed by CDPSs in three steps. Top: transfer of amino acid 1 (phenylalanine) from tRNA to the catalytic S37 residue. Middle: transfer of amino acid 2 (phenylalanine) from tRNA to amino acid 1. Bottom: cyclization. The tetrahedral reaction intermediate at each step is highlighted with a gray background. The chiral carbon center is highlighted (black background).

### DETAILED DESCRIPTION OF THE INVENTION

The present invention concerns a computer-implemented method for ranking and/or identifying substrate-enzyme combinations according to the likelihood that a substrate undergoes catalytic conversion by an enzyme or inhibits the enzyme, and further uses of this method. In particular, said further uses are predicting the substrate range of an enzyme X, screening enzyme variants with altered substrate specificity in relation to a substrate of interest, and predicting the biological phenotype associated with an enzyme X.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation. Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the present context, an "enzyme" is a biological catalyst, typically a protein or a RNA-based catalyst such as a ribozyme, that accelerates chemical reactions by lowering the activation energy. In the context of this application, the term "enzyme" is used interchangeably with "receptor," emphasizing its role as a target for ligands in molecular docking studies.

In the present context, a "substrate" refers to a molecule that undergoes a chemical reaction catalyzed by an enzyme, or that bind to the enzyme and hinder its catalytic activity, the latter also referred to as "inhibitor". In the context of this application, the term "substrate" is used interchangeably with "ligand," representing a broad range of molecules that can interact with the enzyme (receptor) during the molecular docking process. Substrates may include various biomolecules such as proteins, (poly)peptides, metabolites, lipids, DNA, RNA, and small molecules.

"Small molecules" typically refer to organic compounds with relatively low molecular weights and simple structures. As a non-limiting example, small molecules may be defined as having a upper limit of molecular weight of around 900 Daltons. They can include various chemical classes such as drugs, natural products, and synthetic compounds, and they are often used as probes in drug discovery, chemical biology, and other research areas.

A "reaction intermediate" is a molecular entity that is formed and consumed during the course of a chemical reaction. It is a transient species that exists between the reactants and the final products. Reaction intermediates play a crucial role in understanding reaction mechanisms and pathways. These intermediates may be stable or highly reactive, depending on the specific chemical context. In the context of enzymatic reactions, a reaction intermediate often refers to a molecular species formed during the course of the reaction. In some cases, this intermediate may involve a combination of the substrate and the enzyme or a modified form of either. It is a transient state in the overall reaction path before the final products are produced.

A "biological phenotype" in the present context refers to any observable characteristic or trait exhibited by an organism or a population of organisms, such as a human organism, resulting from both genetic factors, such as enzyme sequences, and interactions of such genetic factors and environmental influences. These characteristics may include but are not limited to disease susceptibility, disease progression, response to treatment, biochemical markers, or any other measurable feature relevant to the health, development, or functioning of an organism. In particular in the present context, biological phenotypes may for instance include diagnostic outcomes, disease states (e.g., cancer detection), drug response profiles, or other medically relevant classifications.

In a first aspect, the invention provides a computer-implemented method for ranking and/or identifying substrate-enzyme combinations according to the likelihood that a substrate undergoes catalytic conversion by an enzyme or inhibits the enzyme, the method comprising the following steps:
a) obtaining a 3D structure for each j conformations of one or more enzymes of a same family,
b) obtaining a 3D representation of reaction intermediates of each of i possible substrates of said j conformations in every possible stereochemical configuration z of the reaction intermediate,
c) molecular docking said 3D representations of the reaction intermediates of step b) against each 3D structure of said j conformations of step a), thereby generating a plurality of s docking poses of the substrate in the active site for each izj combination;
d) for each izjs docking pose obtained in step c), deriving a vector of information that represents the docking pose for each of the enzyme with the substrate, the enzyme with the reaction intermediate, and the enzyme with the product of the substrate
e) optionally, where in step d) the vector of information does not comprise interaction energies, converting the vector of information to interaction energies for each of the enzyme with the substrate, the enzyme with the reaction intermediate, and the enzyme with the product of the substrate;
f) training a machine learning model:
   - using a one-step approach, on a training dataset comprising a subset of the i possible substrates and a first subset of the one or more enzymes and the associated likelihoods of each substrate of the subset of substrates undergoing catalytic conversion by the enzymes of the first subset or inhibiting the enzymes of the first subset based on vectors of information as in step d) or interaction energies as in step e), thereby obtaining a machine learning model trained to provide a ranking and/or to identify substrate-enzyme combinations according to the likelihood that a substrate undergoes catalytic conversion by an enzyme or inhibits the enzyme based on the vector of information or interaction energies of each of the enzyme with the substrate, the enzyme with the reaction intermediate, and the enzyme with the product of the substrate,
      or
   - using a two-step approach comprising a first and a second step, the first step comprising training the machine learning model on a training dataset comprising vectors of information as in step d) or interaction energies as in step e), and their associated likelihoods of each substrate undergoing catalytic conversion by the enzymes or inhibiting the enzymes based on their the vector of information or determined interaction energies, and the second step comprising training the machine learning model on a training dataset comprising likelihoods of each substrate undergoing catalytic conversion by the enzymes or inhibiting the enzymes based on their vectors of information or determined interaction energies, and their associated likelihoods of the enzymes providing a certain biological phenotype,
      thereby obtaining a machine learning model trained: to provide a ranking and/or to identify substrate-enzyme combinations according to the likelihood that a substrate undergoes catalytic conversion by an enzyme or inhibits the enzyme based on vectors of information or interaction energies of each the enzyme with the substrate, the enzyme with the reaction intermediate, and the enzyme with the product of the substrate, and
      to provide a likelihood of an enzyme providing a certain biological phenotype according to the likelihood that a substrate undergoes catalytic conversion by an enzyme or inhibits the enzyme based on the vector of information or on interaction energies of each of the enzyme with the substrate, the enzyme with the reaction intermediate, and the enzyme with the product of the substrate; and
g) based on vectors of information and/or interaction energies determined in step d) or e), determining the likelihood that the substrate undergoes catalytic conversion by the enzyme or inhibits the enzyme, using the trained model of step f), and subsequently providing a ranking and/or identifying substrate-enzyme combinations based on said likelihood.

The computer-implemented method of the invention determines a ranking of substrate-enzyme combinations according to the likelihood that a substrate undergoes catalytic conversion by an enzyme or inhibits the enzyme. The present method has the advantage that both the binding (binding strength) or docking of the substrate to the enzyme, i.e. the affinity to the enzyme or receptor, and the efficiency of the reaction, i.e. reaction speed of the conversion of the substrate towards the product, are taken into account to determine this ranking.

Substrates or ligands are usually ranked and/or identified based on a score reflecting their affinity to the enzyme or receptor. However, binding affinity is only one aspect of substrate selection. A substrate not only needs to bind the enzyme, but it must also be able to react in order to be transformed into its cognate product by the enzyme, and thus binding is not sufficient. Docking data alone cannot provide such information on whether a substrate is converted to a product by the enzyme. In order to take this dimension into account, it was chosen to derive, for each docking pose of all possible reaction intermediates, the corresponding pre- and post-reaction pose. Thus, for every possible pose of each ligand in each conformer of an enzyme, the vector of information or interaction energies of 3 configurations are obtained: before or pre reaction (enzyme with the substrate), reaction intermediate, and after or post- reaction (enzyme with the product of the reaction catalyzed by the enzyme). Using the calculated binding energies of these three states, energetically favorable energy paths from substrate to product based on interaction energies can be determined, wherein an "energetically favorable path" is a combination of substrate-intermediate-product poses, whose combined interaction energy profile is globally favorable for converting a substrate into a product (see also further).

For this, a computer-implemented method according to the invention is provided, in which according to a first step a) a 3D structure for each j conformations of one or more enzymes of a same family is obtained.

A group of enzymes is considered to be in the "same family" if they exhibit similar substrate specificity (as assumed or expected based on sequence and/or structure similarity), sharing the ability to catalyze reactions involving a comparable range of substrates. The determination of enzyme family membership can be based on in silico methods, such as sequence homology analysis and structural comparisons, or experimental confirmation of shared substrate specificity. The flexibility of defining enzyme families allows for case-by-case considerations, accommodating variations in sequence, structure, and substrate preference within the established criteria.

An example for considering enzymes to be part of the same family, is as follows for cyclodipeptide synthases (CDPSs): The selection of enzymes is based on (1) sequence similarity (pattern matching), (2) structural similarity (predicted 3D structure), and (3) for the enzymes to be used as in the training/validation datasets, availability of data (ideally, experimental) regarding its substrate specificity. In terms of substrate specificity, the enzymes should use an (any) aminoacyl-tRNA as a substrate, which may be assumed to be the case based on sequence/structure similarity, even in the absence of direct experimental evidence. In the present case, 'similar substrate specificity' is limited to 'is assumed to use tRNA-AA as a substrate'.

A 3D structure for each j conformations of an enzyme preferably relates to one reference 3D structure of an enzyme of interest and additional conformers, i.e. possible alternative conformations the enzyme may have. The 'reference 3D structure' of the enzyme of interest can be obtained from a reference primary sequence or amino acid sequence (for proteins) or nucleotides (for ribozymes) of a reference enzyme of interest, via any method known in the art, such as via algorithms as AlphaFold (in the case of proteins) or experimentally determined such as via X-ray diffraction of crystals, NMR, and more. The additional conformers of the enzyme can be determined, for instance, via clustering of atomic configurations generated from molecular dynamics simulations or any method to sample the conformational space, for instance using the algorithm ClustENMD, or determined using any other method as known in the art. The combination of the reference structure of the enzyme and of the additional conformers, forms the "j conformations" of the enzyme.

Thus, in an embodiment, the 3D structures of the j conformations in step a) include a reference 3D structure obtained from a reference primary amino acid or nucleotide sequence for the enzyme and one or more 3D structures of alternative conformations of the enzyme.

This approach significantly broadens the conformational space that can be explored, thus potentially enhancing the accuracy and efficiency of obtaining an accurate ranking and/or identifying of substrate-enzyme combinations according to the likelihood that a substrate undergoes catalytic conversion by the enzyme or inhibits the enzyme. The reference 3D structure serves as a fundamental model for the enzyme, providing a basis for comparison and analysis. This model is preferably derived from the reference primary amino acid or nucleotide sequence of the enzyme. The 3D structure of this sequence gives a detailed representation of the enzyme's typical conformation, including the spatial arrangement of its amino acids or nucleotides and the configuration of its active site.

The "active site" of an enzyme is a specialized region where substrates bind and undergo catalytic conversion or where inhibitors can bind. It comprises of specific amino acid or nucleotide residues arranged to facilitate substrate recognition, binding, and the catalytic or inhibitory reaction. This site lowers the activation energy required for the reaction or inhibits catalytic activity, enabling enzymes to selectively catalyze specific biochemical reactions or regulate their activity. In embodiments, one enzyme may have more than one active site.

Where an enzyme is composed of multiple subunits, the method treats all subunits as a single unit. If the multimeric enzyme is very large, only the subunits involved in the catalytic site and those affecting the structure of the catalytic subunit are included, and at the very least, only the subunit with the catalytic site should be taken into account.

In addition to the reference 3D structure, the method also employs 3D structures of alternative conformations of the enzyme. These conformations represent variations in the enzyme's structure that may occur under different physiological conditions, in response to various ligands, or in different environments. By considering these alternative conformations, the method can account for the dynamic nature of the enzyme, which may adopt different structures depending on its environment and interactions.

The present method requires that a 3D structure for each j conformations of one or more enzymes of a same family is obtained. This 'one or more' enzymes may refer to at least 1, 2, 3, 4, 5, 6, 7, 8, 9, 10, 15, 20, or more enzymes of the same family, of which said j conformations are obtained.

Preferably, said 'one or more' enzymes includes at least one reference enzyme, a reference for the family of enzymes. Increasing the number of reference enzymes might increase model validity, but will also increase computational requirements. Therefore, a balance is to be found, and this should be determined on a case-by-case basis, also depending on the enzyme family.

A "multistep reaction" refers to a sequential process where the product of each reaction step serves as the substrate for the subsequent reaction step. In this process, an initial enzyme catalyzes the conversion of a substrate into a product. The resulting product then functions as one of the new substrates for the next reaction step, with a new (additional) substrate selected for each subsequent reaction based on experimental or theoretical predictions, independently of previous substrates. This cycle of product formation and subsequent utilization as of substrates continues through multiple reaction steps, forming a chain of reactions. A "single-step reaction" refers to a process where an enzyme catalyzes the conversion of a substrate into a product in a single reaction step. In this process, the enzyme binds to the substrate, facilitating its transformation into the final product without any subsequent steps involving the product as a new substrate. The reaction is completed in one step, with the enzyme returning to its original state and ready to catalyze another substrate molecule.

In the case of multi-step reactions, the product of one step may become the substrate of the next step. In the present method, the method steps b) to g) may thus be repeated, where for step b) substrates are chosen as is done for single-step reactions, or are at least partially based on the previous step (see below). By repeating steps b) to g), a machine-learning model is created and trained for each step of the multi-step reaction. In the case of the CDPS (see Example 4 below), 3 different models are trained, one for each step of the three-step reaction.

Therefore, in an embodiment, method steps b) to g) may be repeated/run again, once or more, such as 1, 2, 3, 4, 5, or more times.

According to step b) of the method, a 3D representation of reaction intermediates of each of i possible substrates of said j conformations in every possible stereochemical configuration z of the reaction intermediate is obtained.

The 'i possible substrates' represent i different potential substrates that can interact with the enzyme.

In the case of multi-step reactions, each preceding reaction of a multi-step reaction acts as a means to reduce the set of potential substrates for the subsequent reaction by eliminating 'poor' substrates with low rankings given by the machine learning model. It is possible to have more than one substrate for the next reaction. This can be exemplified by the CDPS's reactions, which is a multi-step reaction involving three steps and using two substrates at steps 2-3 of the reaction, as further illustrated as Example 4 in the example section.

In the case of multi-step reactions, steps b) to g) of the method are re-run for each sub-step of the multi-step reaction, and for each sub-step (aside from the first step where no previous reaction step occurred), the 'i possible substrates' are a subset constructed from the products derived from the 'i possible substrates' of the previous reaction step, possibly combined with a further set of substrates. Preferably, in a multi-step reaction, the 'i possible substrates' are a subset of the products derived from the 'best' or 'highest ranking' i possible substrates of the previous reaction step, possibly combined with a further set of substrates.

The 'best' or 'highest ranking' i possible substrates refer to enzyme-substrate combinations which rank the highest based on the likelihood of catalytic conversion, as in step g). Based on a predetermined threshold, preferably derived from training data, less promising enzyme-substrate combination may be filtered out, leaving only the 'best' or 'highest ranking' products, which are subsequently used in a next step of a multistep reaction as enzyme. This method facilitates the efficient identification and selection of substrate-enzyme pairs for complex biochemical pathways, ensuring that each step builds upon the previous one to achieve the desired multi-step transformation.

The 'stereochemical configuration z of the reaction intermediate' relates to the z different spatial arrangements or configurations of the reaction intermediates that are exclusively present in the intermediate, and not the substrate and/or product, specifically focusing on stereochemistry, such as the (R) or (S) stereoisomers.

The 3D representations of the reaction intermediates can be determined based on prior knowledge of the enzyme mechanism. Preferably, this prior knowledge is experimentally established knowledge. Alternatively, in case the exact mechanism is not experimentally established, this prior knowledge may be based on one or more suspected mechanism(s) and based on expectations or predictions on how the rection intermediate could look like, preferably based on prior knowledge of similar enzymes.

Enzymes act as catalysts, whose role is to modify and accelerate the rate of a reaction. The lowering of the energy barrier between substrates and products, occurs because of the better stabilization of the intermediate relative to the bound substrates and products. Thus, enzymes have evolved so that they have the highest affinity for their reaction intermediate, whereas substrates and products have a relatively lower affinity. The inventors therefore opted for a docking approach where reaction intermediates are used as docking ligands, rather than substrates and/or products. In the case of reaction intermediates that are covalently bound to the enzyme or to a prosthetic group of the enzyme, this approach also has the advantage of reducing the number of aberrant poses of the ligand in the catalytic site.

In an embodiment where the stereo-configuration of the reaction intermediate is known or where the reaction intermediate does not contain multiple chiral centers compared to the substrate or product, it is or might be sufficient to only provide the a 3D representation of reaction intermediates of each of i possible substrates of said j conformations of the known configuration of the reaction intermediate.

In a subsequent step c) said 3D representations of the reaction intermediates of step b) are molecularly docked against each 3D structure of said j conformations of step a), thereby generating a plurality of s docking poses of the substrate in the active site for each izj combination. Preferably, said docking is performed using a computer system.

In this step, the reaction intermediates of step b), i.e. i substrates x z stereoisomers, are docked against all j conformers of the enzyme of step a). For each izj combination, s docking poses will be obtained, resulting in a total of izjs docking poses. The 'plurality of s docking poses' refers to multiple hypothetical orientations or positions (poses) in which a substrate can dock to the enzyme within a specific conformation and stereochemical configuration, and in particular dock to the enzyme's active site. In an embodiment, the 'plurality of s docking poses' relates to just 1 docking pose, or 2, 3, 4, 5, or more docking poses. This molecular docking can be performed by any tool known in the art. Non-limiting examples of tools may be any tool from the AutoDock suite or derived thereof. Initial poses can be obtained by for instance QuickVina2. The use of multiple conformations for docking allows the method to capture a wider range of potential interactions between the enzyme and the molecules being screened. Each conformation presents a unique 3D structure for docking, offering different potential binding sites and interaction modes for the molecules. By accommodating multiple conformations, the method can detect interactions that might otherwise be overlooked when only a single conformation is considered, and further quantify them. This comprehensive approach enhances the method's capability to identify potential substrates, for instance substrates within biosynthetic gene clusters (BGCs), thereby facilitating the prediction of metabolite structures, or increases the chances of identifying promising candidates for drug design or repurposing.

In an embodiment, the number of alternative conformations used for docking may be 0 i.e. no alternative conformation over the, initial, reference structure, or preferably is at least 1. i.e. the initial, reference, structure and a single additional conformer. The maximum number of alternative conformations is in theory endless, however the more conformations are taken into account, the higher the computational cost. In an embodiment, the number of alternative conformations is between 1 and 1500, preferably between 1 and 1250, or between 1 and 1000, between 1 and 750, between 1 and 500, preferably between 1 and 300, more preferably between 1 and 250, and most preferably between 1 and 200. The latter range provides a good balance between computational efficiency and the coverage of the conformational space. The selection of the conformations can be based on various factors, such as their frequency of occurrence, their relevance to the enzyme's function, or their potential to interact with the molecules being screened.

The substrates and enzymes are composed of residues, such as amino acid residues, nucleotides, fatty acids, and derivatives thereof. Said residues may have side chains, as is generally known. In an embodiment, side chains of selected residues of said enzymes and/or substrates are allowed to be flexible during the molecular docking of step c). Preferably, the molecular docking tool used allows this flexible docking.

'Flexible residues' in the present context refer to specific residues, such as amino acid, nucleotide, or fatty acid residues, in the enzyme or substrate whose conformation can change or adapt during molecular docking simulations. In the docking process, the used software tool allows certain side chains or regions to be flexible, meaning they are not rigidly fixed in a specific conformation but can adjust their orientation and spatial arrangement to accommodate the binding of the substrate to the enzyme. In molecular docking studies, flexibility in certain residues is often considered to account for the natural flexibility of protein structures. It allows the model to explore a range of potential binding poses and better simulate the dynamic nature of molecular interactions between substrates and enzymes. The identification of these flexible residues is often based on structural alignment against reference sequences or other criteria relevant to the specific enzyme being studied. Ideally, all residues would be considered flexible by the used tool, however, this would be at a very high computational cost.

Thus, preferably, said flexible residues are identified by structural alignment against one or more reference sequences, as is known in the art.

Using a single 3D model of the enzyme, as is generally done in the art, neglects the fact that enzymes in solution can adopt different conformations that are in equilibrium with one another. In addition, the transition from the apo (inactive/unbound) state of an enzyme to the ligand-bound complex often implies conformational changes, and therefore, docking ligands to a single, rigid model of the apo state, may score poorly. The present inventors have circumvented this by considering selected side-chain residues of the enzyme and/or substrate as flexible during docking. Such flexible docking methods still consider the backbone of the enzyme as rigid. In addition to flexible docking, the inventors therefore also opted for an approach in which substrates are docked to a set of 3D models, rather than a single 3D model, for each enzyme of interest, circumventing this limitation.

As mentioned, said flexible residues are identified by structural alignment against one or more reference sequences, as is known in the art. These reference sequences used for structural alignment could be derived from a variety of sources including, but not limited to, experimentally determined structures, homology models, or computational predictions. In a most preferred embodiment, the reference sequences are derived from experimentally determined structures. This ensures the highest level of accuracy in the identification of flexible residues.

In step d) of the present method, for each izjs docking pose obtained in step c), a vector of information that represents the docking pose for each of: the enzyme with the substrate, the enzyme with the reaction intermediate, and the enzyme with the product of the substrate, is determined. For this, preferably, for each izjs pose, the reaction intermediates are converted into their corresponding pre-reaction (substrate) configurations, and post-reaction (product) configurations, and local optimization of the docked substrate and product are performed. This local optimization may be performed using tools such as any tool from the AutoDock suite or any tool derived thereof, such as Smina and AutoDock Vina.

A "vector of information" in the context of this method refers to a structured set of data that represents the docking poses obtained in step c) for each of the enzyme with the substrate, the enzyme with the reaction intermediate, and the enzyme with the product of the substrate. It may comprise any type of information, as long as it represent the docking poses of step c).

Examples of such information may for instance include interatomic potentials, interatomic distance, interaction energies, structural descriptors, conformational changes. "Interatomic potentials" represent the potential energy associated with the interactions between atoms in the enzyme-substrate, enzyme-reaction intermediate, and enzyme-product complexes. They provide insights into the stability and strength of interactions within the complex. "Interatomic distances" indicate the distances between atoms within the enzyme-substrate, enzyme-reaction intermediate, and enzyme-product complexes. Changes in these distances can reflect conformational changes or binding events occurring during the reaction. "Interaction energies" quantify the strength of interactions (e.g., hydrogen bonds, electrostatic interactions, van der Waals forces) between the enzyme and the substrate, reaction intermediate, and product. They help assess the binding affinity and specificity of the enzyme for each molecular species involved in the reaction. "Structural descriptors" include geometric parameters, such as bond angles, torsion angles, and dihedral angles, which characterize the spatial arrangement of atoms within the enzyme-substrate, enzyme-reaction intermediate, and enzyme-product complexes. "Conformational changes" describe any structural alterations undergone by the enzyme upon binding to the substrate, reaction intermediate, or product. This may include changes in overall shape, flexibility, or active site configuration.

Preferably, the vector of information of the present method includes interatomic potentials, interatomic distance, or interaction energies, most preferably interaction energies.

According to step e), optionally, where in step d) the vector of information does not comprise interaction energies, the vector of information is converted to interaction energies for each of the enzyme with the substrate, the enzyme with the reaction intermediate, and the enzyme with the product of the substrate.

Substrates are usually ranked and/or identified based on a score reflecting their affinity to the receptor. However, as indicated above, binding affinity is only one aspect of substrate selection. A substrate not only needs to bind the enzyme, but it must also be able to react in order to be transformed into its cognate product by the enzyme: binding is not sufficient. Docking alone cannot provide such information. In order to take this dimension into account, the inventors chose to derive, for each docking pose of all possible reaction intermediates, the corresponding pre- and post-reaction pose. Thus, for every possible pose of each ligand in each conformer of an enzyme, three configurations are obtained: before/pre- reaction, reaction intermediate, and after/post- reaction. Using the vectors of information and/or more preferably calculated interaction energies of these three states, energetically favorable energy paths from substrate to product based on interaction energies can be determined, wherein an "energetically favorable path" is a combination of substrate-intermediate-product poses, whose combined interaction energy profile is globally favorable for converting a substrate into a product. Thus, said three vectors of information or 'interaction energies' or 'energies of interaction' allow capturing both the affinity or binding strength of the reaction intermediate, the substrate and the product, as well as the reactivity or reaction speed. Energetically favorable energy paths from substrate to product are determined based on the vectors of information or interaction energies determined in step d) or e). An 'energetically favorable energy path from substrate to product' is a combination of substrate-intermediate-product poses, whose combined interaction energy profile is globally favorable for converting substrate into product. As it relates to an energy profile that is 'globally' favorable, it does not need to include a pose that is favorable for both binding strength and reaction speed. For instance, it is conceivable that a pose with a medium (or even low) 'binding strength', would be part of a favorable path because its lower 'binding strength' is compensated by a higher 'reactivity'.

According to step g), based on the vectors of information and/or (calculated) interaction energies as determined in steps d) and/or e), the likelihood that the substrate undergoes catalytic conversion by the enzyme or inhibits the enzyme can be determined using a trained machine learning model.

In particular, step g) allows to compare all substrates for any given enzyme in terms of their overall rate of transformation into their cognate product (i.e., considering all conformers and poses (js) for every substrate/stereoisomer (iz) pair).

Subsequently a ranking of substrate-enzyme combinations according to the likelihood that the substrate undergoes catalytic conversion by the enzyme or inhibits the enzyme may be provided, based on/using said trained machine learning model. Said model may be trained as described below, and is preferably as in step f).

Step f) describes training a machine learning model on one or more training dataset. The present method provides two alternative approaches, a one-step approach, and a two-step approach.

Using the 'one-step approach', a machine learning model is trained on a training data set comprising a subset of the i possible substrates and a first subset of the one or more enzymes and the associated likelihoods of each substrate of the subset of substrates undergoing catalytic conversion by the enzymes of the first subset or inhibiting the enzymes of the first subset based on their determined interaction energies and/or vector of information, the latter preferably subsequently converted to interaction energies. This one-step approach results in obtaining a machine learning model trained to provide a ranking and/or to identify substrate-enzyme combinations according to the likelihood that a substrate undergoes catalytic conversion by an enzyme or inhibits the enzyme based on interaction energies and/or vector of information of each of an enzyme with a substrate, the enzyme with an reaction intermediate, and the enzyme with a product of the substrate.

Where reference is made to a "subset" of the one or more enzymes, it is to be understood that, where only one reference enzyme is used, the subset will relate to this one reference enzyme. Where multiple reference enzymes are used, the subset can relate to all of said multiple reference enzymes, or less than all of said multiple reference enzymes. Examples are 1, 2, 3, 4, or more, up to all of said reference enzymes.

Where reference is made to a "subset" of the substrates, it is to be understood that, where only one substrate would be used, the subset will relate to this one substrates. Where multiple substrates are used, which is preferred, the subset can relate to all of said substrates, or less than all of said substrates. Examples are 1, 2, 3, 4, 5, 10, 15, 20, 30, 40, 50, 75, 100, or more, up to all of said reference enzymes.

In an embodiment, the total set of enzymes is divided into subsets for training and/or validating/testing the model.

The substrate-to-product conversion likelihood associated with every substrate of a particular enzyme may not always be available when training and/or validating the model, for instance because the information is unavailable in the literature or cannot be observed experimentally. Therefore in an embodiment, the model may be trained on a subset of substrates as mentioned above, where said subset comprises, preferably only, substrates for which the substrate-to-product likelihood is known. Preferably, the docking steps are performed with all of the substrates, i.e. irrespective of whether the substrate-to-product conversion likelihood is known. Preferably, for the one-step approach, step f) comprises further validating the model on a validation dataset comprising the subset of i possible substrates and a second subset of the one or more enzymes by providing the interaction energies and/or vector of information associated with the combination of substrates and enzymes in said validation dataset and comparing the predicted ranked list with experimentally determined substrates.

In an embodiment, said first and second subset may comprise a different subset of enzymes, or alternative may also comprise the same subset of enzymes.

The 'two-step approach' comprises a first and a second step. The first step comprises training the machine learning model on a training dataset comprising vectors of information as in step d) or interaction energies as in step e), and their associated likelihoods of each substrate undergoing catalytic conversion by the enzymes or inhibiting the enzymes based on their determined interaction energies or vectors of information. The second step comprises training the machine learning model on a training dataset comprising likelihoods of each substrate undergoing catalytic conversion by the enzymes or inhibiting the enzymes based on their determined interaction energies or vector of information, and their associated likelihoods of the enzymes providing a certain biological phenotype.

The first and second step of the two-step approach result in a machine learning model trained:
- to provide a ranking and/or to identify substrate-enzyme combinations according to the likelihood that a substrate undergoes catalytic conversion by an enzyme or inhibits the enzyme based on interaction energies or vector of information of each of an enzyme with a substrate, the enzyme with a reaction intermediate, and the enzyme with a product of the substrate, and
- to provide a likelihood of an enzyme providing a certain biological phenotype according to the likelihood that a substrate undergoes catalytic conversion by an enzyme or inhibits the enzyme on interaction energies or vector of information of each of an enzyme with a substrate, the enzyme with a reaction intermediate, and the enzyme with a product of the substrate.

A "biological phenotype" in the present context refers to any observable characteristic or trait exhibited by an organism or a population of organisms, such as a human organism, resulting from both genetic factors, such as enzyme sequences, and interactions of such genetic factors and environmental influences. These characteristics may include but are not limited to disease susceptibility, disease progression, response to treatment, biochemical markers, or any other measurable feature relevant to the health, development, or functioning of an organism. In particular in the present context, biological phenotypes may for instance include diagnostic outcomes, disease states (e.g., cancer detection), drug response profiles, or other medically relevant classifications determined.

Preferably, for the two-step approach, step f) comprises further validating the machine learning model:
- for the one-step approach, using a validation data set comprising the subset of i possible substrates and a second subset of the one or more enzymes, by providing the interaction energies or vector of information associated with the combinations of substrates and the enzymes in said validation dataset, and comparing the predicted ranked list of substrates with experimentally determined substrates, or
- for the two-step approach, using a validation data set comprising vectors of information as in step d) or interaction energies as in step e), and comparing predicted biological phenotypes with known or experimentally determined phenotypes associated with the enzyme.

In an embodiment, the two steps of the two-step approach may also be combined to one step, preferably where the models of the two step approach are combined and optimized to immediately predict the biological phenotype based on the provided vectors of information as in step d) or interaction energies as in step e). The substrate ranking/likelihood that a substrate undergoes catalytic conversion of inhibits the enzyme is then calculated but not actually provided as an output, which is again used as input in the two-step approach. As the likelihood is calculated but not 'shown', it is preferably possible to extract the likelihood that a substrate undergoes catalytic conversion by an enzyme or inhibits the enzyme, from the model.

In another embodiment, the model could also be trained to predict the biological phenotype, without predicting the substrate ranking/likelihood that a substrate undergoes catalytic conversion of inhibits the enzyme first. In this case the provided vectors of information as in step d) or interaction energies as in step e) are directly used to predict the biological phenotype, without the internal calculation of the likelihood.

The present computer-implemented method thus significantly enhances the prediction accuracy of enzyme-substrate interactions by utilizing multiple 3D protein models and integrating both binding strength and reaction speed. While the method does not provide conventional kcat/KM values in a strict sense, it offers a combined assessment of affinity and reactivity, thereby providing a more comprehensive representation of conformational space and affinity. The present method thus provides a quantitative equivalent for conventional metrics like kcat/KM. "kcat/KM" represents the ratio of an enzyme's catalytic efficiency to its substrate affinity. The method provides a quantitative equivalent to this metric, and uses multiple 3D models, to enhance the accuracy of predicting enzyme-substrate interactions, providing a more comprehensive evaluation of molecular binding and reaction speed. This method's effectiveness is further elevated by considering both the affinity and reactivity of molecules. By docking reaction intermediates instead of free molecules, it better reflects the enzyme's affinity for substrates. Additionally, by deducing pre-and post-reaction conformations for each docked pose, the method assesses molecule reactivity, offering improved methods for predicting metabolite structure, for enzyme variant screening, and for comprehensive evaluation of their potential as drugs or inhibitors. Further, utilizing multiple 3D enzyme structures for docking expands the conformational exploration, enhancing screening exhaustiveness.

In a preferred embodiment of the invention, the computer-implemented method incorporates an advanced process of filtering and/or refining docking poses obtained in step c). This filtering process is preferably based on a comprehensive understanding of the enzyme knowledge, and preferably involves filtering the docking poses based on accurate recognition of atom bonds and/or an empirically determined threshold for the distance between key atoms. This approach ensures that only the most probable and biologically relevant poses are selected for the subsequent steps of the method.

In addition to, or as an alternative to the filtering based on enzyme knowledge, the docking poses may also be refined, preferably by validation against an alternative docking algorithm, or against the same algorithm preferably with different parameters and/or constraints. This additional layer of validation contributes to the robustness of the method by corroborating the results obtained from the primary docking algorithm. In some instances, local re-optimization of poses is performed, to enhance the accuracy of the docking poses. This re-optimization process takes into consideration the local environment of the protein and its potential impact on the docking pose. This dual validation and re-optimization process significantly improves the precision of the docking poses, leading to more reliable interaction energy determinations.

The implementation of this advanced modeling process using multiple 3D models, augmented by the refining of docking poses based on enzyme knowledge and/or an alternative docking algorithm, provides a comprehensive and accurate representation of the biological system. This method enhances our understanding of enzyme behavior under various conformations, which is desired for instance for predicting metabolite structures, for enzyme variant screening, and for the design of effective drugs. By considering a wide range of conformations, the method provides a more encompassing screening of metabolite structures, enzyme variants, and/or potential drugs. The latter, for instance, leads to the identification of drugs that are effective against a broader range of enzyme conformations, increasing the potential therapeutic applications.

In an embodiment, the threshold for the distance between key atoms used in the filtering process will depend on the selected atoms, and should be decided on a case-by-case basis. Pairs of key atoms can be selected in at least 2 different ways: (1) key atoms that should be as far as possible from each other, or (2) key atoms that should be as close as possible from one another.

In another preferred embodiment, the alternative docking algorithm used for validation and/or local re-optimization may be for instance Autodock Suite and tools derived thereof. The selected alternative docking algorithm can be tailored to the specific requirements of the protein and the potential ligand, providing additional flexibility to the method.

As already indicated above, in a preferred embodiment of the computer-implemented method, the one or more enzymes of a same family comprises at least 1 enzyme of the same family, preferably a reference enzyme. This embodiment allows the method to provide a comprehensive evaluation of the conformational space of the enzyme family. As each enzyme in the family can adopt multiple conformations, the use of more of enzymes of the same family, each represented by multiple 3D models, significantly expands the conformational space to be investigated during the docking process. This expansion of the conformational space is a significant advantage over the traditional approach of using a single 3D model for each enzyme. By considering multiple 3D models for each enzyme, the method is able to uncover potential substates, thereby facilitating the prediction of metabolite structure, for enzyme variant screening, and potentially uncover potential drug-protein interactions that may be overlooked when a single 3D model is used. This is because the method is able to consider not only the binding affinity of a substrate to an enzyme, but also the reactivity of reaction, which is reflected in the energy difference between the pre-reaction and intermediate states, and the intermediate and post-reaction states. However, as explained before, it should be noted that the use of multiple enzymes of the same family, each represented by multiple 3D models, increases the computational cost of the method. This is a trade-off for the increased comprehensiveness and robustness of the method. To mitigate this increased computational cost, in a further preferred embodiment, the method may include an initial screening step, in which a subset of the multiple enzymes of the same family is selected based on certain criteria, such as their known or predicted relevance to the pathway, disease or condition being targeted. The docking process is then performed using this subset of enzymes, thereby reducing the computational cost while still benefiting from the advantages of using a multiple enzymes of the same family. In an embodiment, only one enzyme, a reference enzyme, is used, which represents the mode of action of the enzymes of this family of enzymes.

In summary, the method of the present invention enables a more comprehensive exploration of potential substrates, such as substrates within biosynthetic gene clusters (BGCs), thereby facilitating the prediction of metabolite structures, or for variant screening (see below). Such method may also be used to increase the chances of identifying promising potential therapeutic agents. It does this by considering both the affinity and reactivity of a molecule. Affinity refers to the strength of the interaction between a substrate and an enzyme, while reactivity refers to the ability of the substrate to undergo a chemical reaction catalyzed by the enzyme. By taking into account both affinity and reactivity, the method further to what has been discussed above, enhances the chances of finding alternative substrates, by including said alternative mechanisms in the enzyme-substrate ranking. This is particularly beneficial when classical approaches might fall short, such as when the target protein has multiple conformations.

In a second aspect, the invention relates to a computer system for ranking and/or identifying substrate-enzyme combinations according to the likelihood that a substrate undergoes catalytic conversion by an enzyme or inhibits the enzyme, the computer system configured for performing the computer-implemented method as described above in any one of the embodiments.

In a third aspect, the invention relates to a computer program product for ranking and/or identifying substrate-enzyme combinations according to the likelihood that a substrate undergoes catalytic conversion by an enzyme or inhibits the enzyme, the computer program product comprising instructions which, when the computer program product is executed by a computer, cause the computer to carry out the computer-implemented method as described above in any one of the embodiments.

In further aspects, the invention relates to uses of the computer-implemented method as described above in any one of the embodiments, the computer system, and/or the computer program product, for predicting the substrate range of an enzyme X, for screening enzyme variants with altered substrate specificity in relation to a substrate of interest, and/or for predicting the biological phenotype associated with an enzyme X, as will further be described below.

The invention thus provides a computer-implemented method, a computer system and a computer program product for ranking and/or identifying substrate-enzyme combinations according to the likelihood that a substrate undergoes catalytic conversion by an enzyme or inhibits the enzyme, as well uses of the method, computer system and/or the computer program product, and computer-implemented methods of predicting substrate-range of an enzyme X, of screening enzyme variants with altered substrate specificity in relation to a substrate of interest, and for predicting the biological phenotype associated with an enzyme X. A person having ordinary skill in the art will appreciate that the method may be implemented in the computer program product and/or executed using the computer system. It is also clear for a person having ordinary skill in the art that the ranking of substrate-enzyme combinations according to the likelihood that a substrate undergoes catalytic conversion by an enzyme or inhibits the enzyme can be used for predicting the substrate range of an enzyme X, for screening enzyme variants with altered substrate specificity in relation to a substrate of interest, and/or for predicting the biological phenotype associated with an enzyme X. It is thus clear that embodiments and related advantages described for only one of the aspects, equally relate to the further aspects of the invention, and that the various aspect thus should be considered together.

The invention thus also relates to the use of the computer-implemented method, computer system and/or computer program product for predicting the substrate range of an enzyme X. Likewise, the invention also relates to a computer-implemented method of predicting the substrate range of an enzyme X using the computer-implemented method as described above in any of the embodiments, comprising the steps of:
i) for said enzyme X, obtaining the vectors of information or interaction energies of each of enzyme X with the substrate, the enzyme X with the reaction intermediate, and the enzyme X with the product of the substrate, for each izjs pose of enzyme X as in steps a) to d) or a) to e) of the computer-implemented method for ranking and/or identifying substrate-enzyme combinations according to the likelihood that a substrate undergoes catalytic conversion by an enzyme or inhibits the enzyme, and
ii) using said vectors of information or interaction energies obtained in i), predicting the substrate range of enzyme X using the machine learning model of step f), preferably using the one-step approach.

Said steps i) and ii) a described further below, may also relate to the use for predicting the substrate range of an enzyme X. In particular, according to step i):
- First, a 3D structure for each j conformations of enzyme X is obtained similar as in step a) described above, starting from the primary sequence of said enzyme X.
- Based on knowledge of the mechanisms of enzyme X, subsequently 3D representations of reaction intermediates of each of i possible substrates of said j conformations in every possible stereochemical configuration z of the reaction intermediate are obtained, similar as in step b) described above.
- Said 3D representations of the reaction intermediates are docked against each 3D structure of said j conformations, thereby generating a plurality of s docking poses of the substrate in the active site for each izj combination.
- The izjs docking poses obtained are optionally refined using an alternative docking algorithm and/or filtered based on enzyme knowledge as described above.
- Similar as for step d) of the computer-implemented method, for each izjs docking pose, or optionally a refined and/or filtered subset of all of said izjs docking poses, the vector of information representing the docking pose of each of the enzyme with the substrate, the enzyme with the reaction intermediate, and the enzyme with the product of the substrate are then determined, and
- Similar as for optional step e) where the vector of information does not comprise interaction energies, converting the vector of information to interaction energies for each of the enzyme with the substrate, the enzyme with the reaction intermediate, and the enzyme with the product of the substrate.

In particular, according to step ii):
- using said vector of information or interaction energies of each of the enzyme with the substrate, the enzyme with the reaction intermediate, and the enzyme with the product of the substrate obtained in i), the substrate range of enzyme X is predicted using the machine learning model of described in step f) above, preferably using the one-step approach, said machine learning model which ranks substrate-enzyme combinations according to the likelihood that a substrate undergoes catalytic conversion by an enzyme or inhibits the enzyme.

Preferably, said vector of information or said three interaction energies of the enzyme with the substrate, the enzyme with the reaction intermediate, and the enzyme with the product of the substrate, is the only data supplemented to the machine learning model to predict the substrate range of enzyme X.

The knowledge referred to in steps i) and ii) preferably is experimentally established knowledge. Alternatively, in case the exact mechanism is not experimentally established, this prior knowledge may be based on one or more suspected mechanism(s) and based on expectations or predictions.

The advantage of this computer-implemented method is the ability to understand, calculate, estimate, and/or use the vector of information or interaction energies between enzyme X and different substrates, intermediates, and products. This understanding allows for an accurate prediction of the substrate range of the enzyme, based on both the affinity or binding strength of the reaction intermediate, as well as the reactivity or reaction speed of enzyme X and its substrates. The prediction of the substrate range of an enzyme holds significant implications for its effective use in various applications. This can have far-reaching impacts across numerous sectors such as the chemical, pharmaceutical, and bio-energy industries. It allows for the maximization of the enzyme's potential by identifying its full range of substrates. This can lead to the discovery of new applications for the enzyme, thereby broadening its utility and value.

In an embodiment, the method involves using the obtained vectors of information or interaction energies to predict the substrate range of enzyme X using a machine learning model. The machine learning model serves as a powerful tool for interpreting the vectors of information or interaction energies and making accurate predictions. In an embodiment, the machine learning model is a deep learning model, and most preferably, it is regression model. In essence, any type of supervised machine leaning method could be used, when adapted to the purpose of the present method. The use of such advanced machine learning models can significantly enhance the accuracy and reliability of the predictions, thereby improving the effectiveness and utility of the method.

Thus, the preferred embodiments of the invention provide a comprehensive and effective method for predicting the substrate range of an enzyme. The method leverages the power of machine learning and the understanding of the vectors of information or interaction energies to make accurate predictions. This can lead to the discovery of new applications for enzymes, thereby benefiting various sectors such as the chemical, pharmaceutical, and bio-energy industries.

As briefly mentioned above, in an aspect the invention also relates to the use of the computer-implemented method, computer system and/or computer program product for screening enzyme variants with altered substrate specificity and/or catalytic activity in relation to a substrate of interest. Likewise, the invention also relates to a computer-implemented method for screening enzyme variants with altered substrate specificity and/or catalytic activity in relation to a substrate of interest using the computer-implemented method as described above in any of the embodiments, comprising the steps of:
i) for a plurality, or one or more, of said enzyme variants and a reference enzyme, obtaining the vector of information or interaction energies of each of the enzyme variants with the substrate, the enzyme variants with the reaction intermediate, and the enzyme variants with the product of the substrate, for each izjs pose as in steps a) to d) or a) to e) of the computer-implemented method for ranking and/or identifying substrate-enzyme combinations according to the likelihood that a substrate undergoes catalytic conversion by an enzyme or inhibits the enzyme, and
ii) using said vector of information or interaction energies obtained in i), to identify an enzyme variant having an altered substrate specificity and/or catalytic activity compared to the reference enzyme using the machine learning model of step f), preferably using the one-step approach.

Said steps i) and ii) as described further below, may also relate to the use for screening enzyme variants with altered substrate specificity and/or catalytic activity in relation to a substrate of interest.

"Enzyme variants with altered substrate specificity" refer to modified forms of an enzyme that exhibit changes in their preference for interacting with different substrates. In other words, these are variations or mutants of an enzyme that have been engineered or naturally evolved to recognize and catalyze reactions with substrates different from those typically accepted by the wild-type (natural) enzyme, in the present context referred to as the 'reference enzyme'. The substrate specificity of an enzyme is determined by the shape, size, and chemical properties of its active site. None limiting examples are changes in the amino acid or nucleotide sequence of the enzyme, either through genetic modification or natural mutations, which can lead to alterations in the active site, resulting in a shift in substrate specificity. Preferably, the enzyme variants comprise active sites that differ from one to another by at least one mutation in their amino acid or nucleotide sequence. These modified/variant enzymes can have practical applications in various fields. In biotechnology and industrial processes, researchers may engineer enzyme variants to enhance their performance or enable them to work with a broader range of substrates. In the context of drug development, understanding and manipulating substrate specificity can be crucial for designing enzymes that target specific biochemical pathways, interact with particular drug compounds, or for designing enzyme inhibitors, or for predicting metabolite structure.

"Enzyme variants with altered catalytic activity" refer to modified forms of an enzyme that demonstrate changes in their efficiency or rate of catalysis compared to the wild-type (natural) enzyme, referred to as the 'reference enzyme'. These variants may exhibit either an increase or decrease in their catalytic activity towards a given substrate, without necessarily altering their preference for interacting with different substrates. Alterations in catalytic activity can arise from modifications in the enzyme's active site, as well as changes in its structural dynamics or conformational flexibility. Such modifications may result from genetic engineering techniques or naturally occurring mutations in the enzyme's amino acid or nucleotide sequence. Enzyme variants with altered catalytic activity hold significant implications across various fields. In biotechnology and industrial processes, they can be utilized to optimize enzymatic reactions, enhance production yields, or tailor specific chemical transformations. In drug development, understanding and manipulating catalytic activity is essential for designing enzymes with tailored pharmacokinetic properties, improving drug metabolism, or developing enzyme-based therapies. Additionally, enzyme variants with altered catalytic activity can provide insights into enzyme evolution, structure-function relationships, and mechanisms of enzymatic catalysis.

In particular, according to step i):
- First, a 3D structure for each j conformations of a plurality of enzyme variants and a reference enzyme is obtained similar as in step a) described above, starting from the primary sequences of said enzyme of interest and enzyme variants.
- Based on knowledge of the mechanisms of the reference enzyme and/or of said enzyme variants, subsequently 3D representations of reaction intermediates of each of i possible substrates of said j conformations in every possible stereochemical configuration z of the reaction intermediate are obtained, similar as in step b) described above. Preferably, this i possible substrates includes the substrate of interest.

- Said 3D representations of the reaction intermediates are docked against each 3D structure of said j conformations, thereby generating a plurality of s docking poses of the substrate in the active site for each izj combination.
- The izjs docking poses obtained are optionally refined using an alternative docking algorithm and/or filtered based on enzyme knowledge as described above.
- Similar as for step d), for each izjs docking pose, or optionally a refined and/or filtered subset of all of said izjs docking poses, the vector of information representing the docking pose of each of the enzyme with the substrate, the enzyme with the reaction intermediate, and the enzyme with the product of the substrate are then determined, and
- Similar as for optional step e) where the vector of information does not comprise interaction energies, converting the vector of information to interaction energies for each of the enzyme with the substrate, the enzyme with the reaction intermediate, and the enzyme with the product of the substrate.

In particular, according to step ii):
- using said vector of information or interaction energies of each of the reference enzyme and enzyme variants with the substrate, identify an enzyme variant having an altered substrate specificity and/or catalytic activity compared to the reference enzyme using the machine learning model of described in step f) above, preferably using the one-step approach, said machine learning model which ranks substrate-enzyme combinations according to the likelihood that a substrate undergoes catalytic conversion by an enzyme or inhibits the enzyme.

Preferably, said vector of information or three interaction energies of the enzymes with the substrate, the enzymes with the reaction intermediate, and the enzymes with the product of the substrate, is the only data supplemented to the machine learning model to identify enzyme variants having an altered substrate specificity and/or catalytic activity compared to the reference enzyme.

The primary advantage of this embodiment is the enhanced efficiency of in silico screening of modified enzyme variants and determination of their substrate range or preference. This is a significant improvement over prior art methods that typically involve docking all possible substrates or inhibitors to a single 3D model of each protein, ribozyme, or enzyme. The present embodiment, on the other hand, uses multiple 3D models of the enzyme, thereby providing a better representation of the conformational space for the target enzyme. This allows for a more accurate and comprehensive screening of enzyme variants, thereby facilitating the identification of those with altered substrate specificity and/or catalytic activity.

In a further preferred embodiment, the machine learning model used in step f) of the first aspect may be trained using a training set comprising vectors of information representing the docking poses and/or interaction energies obtained from one or more known enzyme-substrate pairs. This ensures that the machine learning model is well-equipped to accurately identify enzyme variants with altered substrate specificity and/or catalytic activity.

In a preferred embodiment of the invention, the computer-implemented method enhances the identification of the altered substrate specificity and/or catalytic activity in relation to a substrate of interest. This alteration includes an increase in specificity, affinity, and/or catalytic activity of the enzyme variant compared to the reference enzyme. It is worth noting that the altered substrate specificity is not limited to higher specificity, affinity, and/or catalytic efficiency. In another preferred embodiment, the altered substrate specificity could also include a similar or even lower specificity, affinity, and/or catalytic efficiency of the enzyme variant compared to the reference enzyme. Lower reactivity and/or affinity for a given substrate could for instance be beneficial to limit side-reactions catalyzed by an enzyme on a non-desired substrate that would be a typical contaminant present together with the desired substrate. This would reduce by-product formation, but also potentially reduce the amount of enzyme needed, as it would no longer be (or less) saturated or occupied by the presence of the undesired substrate.

In an embodiment, step ii) includes:
- predicting the substrate range and/or catalytic activity of the reference enzyme and of the enzyme variants based on the vector of information representing the docking poses or interaction energies of the enzymes with the substrate, the enzymes with the reaction intermediate, and the enzymes with the product of the substrate,
- comparing the predicted substrate ranges and/or catalytic activity of the reference enzyme with those of the enzyme variants, and
- identifying an enzyme variant having an altered substrate specificity and/or catalytic activity compared to the reference enzyme.

In a further preferred embodiment, the substrates range includes the substrate of interest.

Additionally, the degree of alteration in substrate specificity and/or catalytic activity is not fixed and can be adjusted according to the requirements of the specific drug discovery or design project. In a more preferred embodiment, the degree of increase or decrease in specificity, affinity, and/or catalytic efficiency could range from at least 10% to at least 100%, such as at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95%, compared to the reference enzyme. Alternatively, a fold increase or decrease of 2, 4, 5, 8, 10, 12, 15, 20, 25, 40, 50, or more can obtained. This level of flexibility and adjustability in the alteration of substrate specificity and/or catalytic activity provides a significant advantage in the application of the method in various scenarios of drug discovery and design. It allows for the customization of the method according to the specific requirements and constraints of the project, thereby enhancing the effectiveness and efficiency of the process.

In a preferred embodiment, the steps are repeated to screen for enzyme variants with altered substrate specificity and/or catalytic activity in relation with a plurality of substrates of interest. The embodiment involves the repetition of the docking process for multiple substrates. This repetition ensures a more exhaustive screening of the substrates, thereby increasing the probability of identifying the most potent substrates for the most interesting enzyme variant.

In another aspect, the invention relates to a use of the computer-implemented method, computer system and/or computer program product, preferably wherein the machine learning model is trained using the two-step approach, for predicting the biological phenotype associated with an enzyme X. Likewise, the invention also relates to a computer-implemented method of predicting the biological phenotype associated with an enzyme X, using the computer-implemented method as described above in any of the embodiments, preferably wherein the machine learning model is trained using the two-step approach, comprising the steps of:
i. for said enzyme X, obtaining the vectors of information representing the docking pose or interaction energies of each of enzyme X with the substrate, the enzyme X with the reaction intermediate, and the enzyme X with the product of the substrate, for each izjs pose of enzyme X as in steps a) to d) or a) to e) of the computer-implemented method for ranking and/or identifying substrate enzyme combinations described above, and
ii. using said vectors of information or interaction energies obtained in i), to predict biological phenotype associated with enzyme X using the machine learning model of step f), preferably using the two-step approach.

In particular, according to step i):
- First, a 3D structure for each j conformations of enzyme X is obtained similar as in step a) described above, starting from the primary sequence of said enzyme X.
- Based on knowledge of the mechanisms of enzyme X, subsequently 3D representations of reaction intermediates of each of i possible substrates of said j conformations in every possible stereochemical configuration z of the reaction intermediate are obtained, similar as in step b) described above.
- Said 3D representations of the reaction intermediates are docked against each 3D structure of said j conformations, thereby generating a plurality of s docking poses of the substrate in the active site for each izj combination.
- The izjs docking poses obtained are optionally refined using an alternative docking algorithm and/or filtered based on enzyme knowledge as described above.
- Similar as for step d) of the computer-implemented method, for each izjs docking pose, or optionally a refined and/or filtered subset of all of said izjs docking poses, the vector of information representing the docking pose of each of the enzyme with the substrate, the enzyme with the reaction intermediate, and the enzyme with the product of the substrate are then determined, and
- Similar as for optional step e) where the vector of information does not comprise interaction energies, converting the vector of information to interaction energies for each of the enzyme with the substrate, the enzyme with the reaction intermediate, and the enzyme with the product of the substrate.

In particular, according to step ii):
- using said vectors of information or interaction energies of each of the enzyme with the substrate, the enzyme with the reaction intermediate, and the enzyme with the product of the substrate obtained in i), the biological phenotype associated with enzyme X is predicted using the machine learning model of step f) above, preferably using the two-step approach, said model which preferably provides a likelihood that an enzyme provides a certain biological phenotype according to the likelihood that a substrate undergoes catalytic conversion by an enzyme or inhibits the enzyme.

Preferably, said vector of information or said three interaction energies of the enzyme with the substrate, the enzyme with the reaction intermediate, and the enzyme with the product of the substrate, is the only data supplemented to the machine learning model to predict the biological phenotype associated with enzyme X.

Preferably, the machine learning model is trained using the two step approach as described above. Preferably, primary sequences of (known) variant enzymes of which the enzyme substrate spectrum has been characterized previously, and their associated biological phenotypes, have been used to train the machine learning model.

The knowledge referred to in steps i) and ii) preferably is experimentally established knowledge. Alternatively, in case the exact mechanism is not experimentally established, this prior knowledge may be based on one or more suspected mechanism(s) and based on expectations or predictions.

The disclosed method and use for predicting biological phenotypes offers several advantages. Firstly, it provides a comprehensive representation of enzyme-substrate interactions through the utilization of a vector of information or interaction energies derived from docking poses. This approach captures the intricate molecular interactions underlying enzymatic function, enhancing the understanding of biochemical processes. Additionally, the method integrates mechanistic knowledge of enzyme-substrate interactions, incorporating experimentally established or suspected mechanisms. This ensures that the predictive models account for the underlying biological processes driving the observed phenotypes. Furthermore, the method integrates machine learning techniques, leveraging a two-step approach to capture complex relationships between enzyme characteristics and biological phenotypes. This integration enhances predictive accuracy and robustness, making the method suitable for various clinical and research applications. Lastly, the method's ability to predict diverse biological phenotypes, including diagnostic outcomes, disease states, and drug response profiles, holds significant clinical relevance. It can potentially inform personalized medicine approaches by guiding treatment decisions based on individual patient characteristics. Overall, the disclosed method offers a powerful and versatile tool for predicting biological phenotypes, with implications for both research and clinical practice.

The invention is further described by the following non-limiting examples which further illustrate the invention, and are not intended to, nor should they be interpreted to, limit the scope of the invention.

### EXAMPLES AND DESCRIPTION OF FIGURES

The present invention will now be further exemplified with reference to the following examples. The present invention is in no way limited to the given examples or to the embodiments presented in the figures. The following description of the figures of specific embodiments of the invention is merely exemplary in nature and is not intended to limit the present teachings, their application or uses.

### Model enzyme and reaction used for the development of the workflow

The cyclodipeptide synthase (CDPS) family of proteins may be used as a model for developing the substrate prediction workflow according to the invention. This family of enzymes produces cyclodipeptides (CDPs) in a 3-step reaction: the first and second amino acids are sequentially transferred from their tRNA to the catalytic serine residue in steps 1 and 2, respectively, and the dipeptide undergoes self-cyclization in step 3, resulting in the release of a free cyclic dipeptide from the catalytic serine residue, as shown in Figure 1.

All steps involve the formation of a tetrahedral reaction intermediate covalently bound to the catalytic serine residue (S37; AlbC numbering) (Figure 2).

In the scope of developing a prediction model for the substrate range of CDPS enzymes as described in the examples below, the focus lays on step 1 of the CDPS reaction (i.e. transfer of amino acid 1 from its tRNA to the catalytic serine residue). For each example sets 1, 2, and 3 described below, three versions are proposed:
a) A version using ensemble docking of the intermediate, but without splitting of the docked intermediate into pre- (or post-) reaction pose. This version combines ensemble docking, where ligands are docked to a set of 3D models instead as a single 3D model (new vs. conventional); and docking of the reaction intermediate instead of ligand (new vs. conventional). This version allows comparing the affinity of ligands at the reaction intermediate step, but neglects the binding affinity of the free ligand to the receptor protein. This version is termed 'basic'.
b) The version in a), with splitting of the docked intermediate into its corresponding pre-reaction pose only. With this additional step, the free ligands can be compared between each other in terms of binding affinity for the receptor protein, as would be done with a traditional (ensemble) docking approach, except that the obtained poses are expected to reflect both binding affinity and correct positioning of the ligand so that the reaction can proceed. This version is termed 'intermediate'.
c) The version in a), with splitting of the docked intermediate into its corresponding pre-and post-reaction poses, calculation of the corresponding energy paths, and simulated kinetic competition between all possible substrates, conformers, and poses. This version allows ranking and/or identifying the ligands based on both their affinity for the protein and their propensity to be converted into their cognate product by the enzyme. This version is termed 'full'.

Version c (full) is the version that corresponds to the present invention. Versions a (basic) and b (intermediate) serve to break down the method in simpler steps thereby explaining version c (full) and its advantages.

### Example set 1 - Prediction of substrate range for an enzyme

### Context

Determine the substrate range/preference of an unknown (uncharacterized) enzyme that belongs to a known family.

A 'family' was described above in the text, and may for instance correspond to a group of enzymes of which the catalytic mechanism is known and there are already characterized members of the group in terms of substrate spectrum. Note that the concept of family here is flexible and should be defined on a case-by-case basis. Similarly, the catalytic mechanism could be 'suspected', rather than proven experimentally, and the substrate spectrum could be 'predicted' using alternative in silico methods, rather than determined experimentally.

### Example 1a (basic)

1. Retrieve primary sequences of enzymes for which the substrate spectrum has been previously characterized (known enzymes). Also retrieve the corresponding substrate spectra, optionally extended by experimentally determining the substrate spectrum of yet uncharacterized enzymes.
2. Process 'known' enzyme sequences (collected at step 1) as follows:
   a) For each primary sequence, **obtain one reference 3D model** of the enzyme. This can for instance be done using algorithms such as AlphaFold. Alternatively, experimentally determined structures can be used (X-ray diffraction of crystals, NMR etc.).
   b) For each primary sequence, derive additional conformers, i.e. possible alternative conformations of the enzyme, in order to **generate an ensemble of *j* conformers,** which includes the reference 3D model from step 2a. The conformer ensemble can be generated using algorithm ClustENMD.
   c) Based on the knowledge of the enzyme mechanism, determine the structure of the reaction intermediate, and **obtain 3D models of the reaction intermediate(s) corresponding to each of the possible substrates *i* to be evaluated, in every possible stereochemical configuration *z*.** For instance, in the case of step 1 of the cyclodipeptide synthetase (CDPS) reaction, the reaction intermediate is a tetrahedral intermediate bound to the catalytic serine residue of the enzyme, with a tRNA moiety (invariable), and an amino acid moiety (variable substrate). This intermediate can, in theory, exist either in the (R) or (S) configuration - configuration not determined for this reaction: if the configuration is known, then one can decide to only use the expected stereoisomer.
   d) Perform molecular docking for all reaction intermediates of step 2c (*i* substrates x *z* stereoisomers), against all *j* conformers of step 2b, for every enzyme, in order to **generate s docking poses for each *izj* combination,** for each enzyme. For docking, preferably allow side-chains of selected residues to be considered flexible. Docking can be performed with tools from the AutoDock suite, or any other docking algorithm. QuickVina2 can be used to obtain the initial poses. Flexible residues can be identified by structural alignment against one or more reference sequence(s), for instance. For example, for CDPS step 1, the side-chains of all reported residues from the P1 and P2 pockets, as well as those of the catalytically relevant residues, are identified by structural alignment vs. the well-characterized AlbC protein, and are allowed to be flexible during docking.
   e) Optionally, **filter docked poses based on enzyme knowledge** (inter-atomic distances, specific angles/torsions etc.) in order to remove aberrant poses. For instance, poses can be based on (i) correct recognition of (non-explicit) atom bonds by standard chemo-informatics tools (openbabel) and (ii) an empirically determined threshold for the distance between 2 key atoms (1 from a side-chain of the receptor and 1 from the ligand).
   f) Optionally, **refine docked poses** using an alternative docking algorithm. For instance, a local re-optimization of poses can be performed using Smina (a fork of AutoDock Vina).
   g) **For each *izjs* pose, derive a vector of information describing the reaction intermediate-enzyme complex.** Descriptors in the vector can contain for instance inter-atomic distances between key atoms of the ligand and enzyme, inter-atomic potentials, and any score that reflects the overall affinity of the ligand to the enzyme. They can include the energy of interaction between the ligand (in its reaction intermediate form) and the enzyme. Preferably and according to the present invention, focus on the energy of interaction as the sole descriptor. For reaction intermediates that are not covalently bound to the enzyme or to a prosthetic group of the enzyme, this is readily accessible. However, for reaction intermediates that are covalently attached to the enzyme - as is the case for step 1 of the CDPS reaction - we consider the covalently-attached reaction intermediate as a flexible side-chain of the enzyme. Therefore, we have developed an approach that calculates this interaction energy from (i) the complete system (covalently-attached reaction intermediate and flexible side-chains), (ii) the complete system, but without flexible side-chains, and (iii) the complete system, but without the covalently-attached reaction intermediate.
3. Create a predictive model based on 'known' enzymes (collected at step 1):
   a) Divide the primary sequences (collected at step 1) into a training dataset and a validation dataset.
   b) Use any linear regression machine-learning model to develop and calibrate a model that ranks possible ligands according to their probability of being used as substrates by each of the known enzymes in the training dataset, based on their associated vectors of information (from step 2g) and known substrate spectrum (collected at step 1). The idea would be to initially use a simple model based on a linear combination of the terms in the vectors of information (typically, at least the interaction energy), possibly after transformation (log, ...) - however, this idea shifted based on the preference for version c (full) as described below.
   c) Validate the model developed at step 3b with the validation dataset, using only their associated vectors of information (from step 2g) as an input, and comparing the predicted ranked list of ligands with the experimentally determined ligands.
   d) Optionally, repeat steps 3a-3c iteratively with different machine-learning models and/or different training/validation datasets and/or different initial parameter values, and compare the predictions of the different models to select the best model.
**4. Determine the substrate range of the unknown enzyme:**
   a) Retrieve the primary sequence of interest.
   b) Derive a vector of information for the unknown enzyme by applying steps 2a-2g.
   c) Predict the substrate range by applying the predictive model developed at steps 3a-3d, using the vector of information (from step 4b) as a sole input.

### Example 1b (intermediate)

1. Retrieve primary sequences of enzymes for which the substrate spectrum has been previously characterized (known enzymes). Also retrieve the corresponding substrate spectra. Optionally extended by experimentally determining the substrate spectrum of yet uncharacterized enzymes.
2. Process 'known' enzyme sequences (collected at step 1) as follows:
   a) to f) as in example 1a
   g) **For each *izjs* pose, convert the reaction intermediate into its corresponding pre-reaction (substrate) configuration, and perform local optimization of the docked ligand (substrate).** For instance, conversion of the reaction intermediate to the corresponding substrate configuration can be done using home-made scripts, and local optimization can be performed using Smina and AutoDock Vina, with a heuristic developed in-house for the choice of the best optimized pose.
   h) **For each *izjs* pose, derive a vector of information describing the substrate-enzyme complex.** Descriptors in the vector can contain for instance inter-atomic distances between key atoms of the ligand and enzyme, inter-atomic potentials, and any score that reflects the overall affinity of the ligand to the enzyme. They can include the energy of interaction between the substrate and the enzyme.
      Preferably according to the present invention, focus on the energy of interaction as the sole descriptor. In the case of substrate-enzyme complexes, the ligand is not covalently bound to the enzyme or to a prosthetic group of the enzyme, and the interaction energy is thus readily accessible.
3. Create a predictive model based on 'known' enzymes (collected at step 1):
   a) Divide the primary sequences (collected at step 1) into a training dataset and a validation dataset.
   b) Use any machine-learning model to develop and calibrate a model that ranks possible ligands according to their probability of being used as substrates by each of the known enzymes in the training dataset, based on their associated vectors of information (from step 2h) and known substrate spectrum (collected at step 1).
      The idea would be to initially use a simple model based on a linear combination of the terms in the vectors of information (typically, at least the interaction energy), possibly after transformation (log, ...) - however, this idea shifted based on the preference for version c (full) as described below.
   c) Validate the model developed at step 3b with the validation dataset, using only their associated vectors of information (from step 2h) as an input, and comparing the predicted ranked list of ligands with the experimentally determined ligands.
   d) Optionally, repeat steps 3a-3c iteratively with different machine-learning models and/or different training/validation datasets and/or different initial parameter values, and compare the predictions of the different models to select the best model.
**4. Determine the substrate range of the unknown enzyme:**
   a) Retrieve the primary sequence of interest.
   b) Derive a vector of information for the unknown enzyme by applying steps 2a-2h.
   c) Predict the substrate range by applying the predictive model developed at steps 3a-3d, using the vector of information (from step 4b) as a sole input.

### Example 1c (full)

1. Retrieve primary sequences of enzymes for which the substrate spectrum has been previously characterized (known enzymes). Also retrieve the corresponding substrate spectra. Optionally extended by experimentally determining the substrate spectrum of yet uncharacterized enzymes.
2. Process 'known' enzyme sequences (collected at step 1) as follows:
   a) to f) as in example 1a
   g) **For each *izjs* pose, convert the reaction intermediate into the corresponding pre- (substrate) and post-reaction (product) configurations, and perform local optimization of the docked substrate and product.** For instance, conversion of the reaction intermediate to the corresponding substrate configuration can be done using home-made scripts, and local optimization can be performed using Smina and AutoDock Vina, with a heuristic developed in-house for the choice of the best optimized pose.
   h) **For each *izjs* pose, calculate the energy of interaction for the substrate-enzyme, reaction intermediate-enzyme, and product-enzyme complexes.** For ligands that are not covalently bound to the enzyme or to a prosthetic group of the enzyme, this is readily accessible. However, for ligands that are covalently attached to the enzyme - as is the case for the reaction intermediate and product of step 1 of the CDPS reaction -the covalently-attached ligand are considered as a flexible side-chain of the enzyme. Therefore, we have developed an approach that calculates this interaction energy from (i) the complete system (covalently-attached ligand and flexible side-chains), (ii) the complete system, but without flexible side-chains, and (iii) the complete system, but without the covalently-attached ligand.
3. Create a predictive model based on 'known' enzymes (collected at step 1):
   a) Divide the primary sequences (collected at step 1) into a training dataset and a validation dataset.
   b) Develop a kinetic mechanistic model that uses the 3 interaction energies of the *izjs* poses (from step 2h) to simulate the time-dependent conversion of substrates to products. At minimum, the model is based on a biochemical reaction network describing, for each *izjs* pose, (i) binding of the substrate to the enzyme, (ii) conversion of the substrate into the reaction intermediate, and (iii) conversion of the reaction intermediate to the product. Reaction rates and equilibrium constants in the model are based on the difference in interaction energies between the different molecular species (substrate, reaction intermediate, product). The network can optionally include exchange reactions between different poses and/or conformers of the same ligand, at the substrate, reaction intermediate, or product stage; at the reaction intermediate stage, exchange reactions should be further restricted to exchange between identical stereoisomers.
   c) Develop a simulation framework to simulate the behavior of the kinetic model (from step 3b). More specifically, this framework should allow comparing all ligands for any given enzyme, in terms of their overall rate of transformation into their cognate product (i.e. considering all conformers and poses (*js*) for every substrate/stereoisomer (iz) pair). Ideally - but not necessarily - the simulation framework should take into account possible differences in concentrations between different substrates. The simulation framework that we is developing comprises performing stochastic simulations - either with all substrates together or with overlapping subsets of at least 2 different substrates - in order to determine a quantitative ranking of substrates for every enzyme.
   d) Calibrate the model parameters of step 3b with the training dataset, using the simulation framework of step 3c. This step consists in optimizing parameter values so that the input data (interaction energies for the substrate-enzyme, reaction intermediate-enzyme, and product-enzyme complexes; from step 2h) result in substrate ranking predictions as close as possible to the known substrate spectrum of the enzymes in the training dataset. For this, develop an objective function that combines the predicted vs. experimental relative substrate preference (qualitative ranking) and predicted vs. experimental absolute substrate preference (quantitative probability of selecting each substrate). Any minimization algorithm can be used to minimize this objective function by altering the parameter values in the kinetic model.
   e) Validate the model calibrated at step 3d with the validation dataset, using only their interaction energies for the substrate-enzyme, reaction intermediate-enzyme, and product-enzyme complexes (from step 2h) as an input, and comparing the predicted ranked list of substrates with the experimentally determined substrates. This will typically be performed using the same objective function as in step 3d.
   f) Optionally, repeat steps 3a-3e iteratively with different models (step 3b) and/or different training/validation datasets (steps 3a and 3e) and/or different simulation frameworks (step 3c) and/or different initial parameter values (step 3d) and/or different objective functions (step 3d), and compare the predictions of the different models to select the best model. Preferably test variations of (i) the kinetic model (for instance, with or without inter-conformer exchanges), (ii) the simulation framework (for instance, simulations with all possible substrates at the same time vs. successive, overlapping 2-by-2 competitions), and (iii) the objective function (from fully quantitative ranking to semi-quantitative ranking).
4. Determine the substrate range of the unknown enzyme:
   a) Retrieve the primary sequence of interest.
   b) Derive interaction energies for the substrate-enzyme, reaction intermediate-enzyme, and product-enzyme complexes, for the unknown enzyme, by applying steps 2a-2h.
   c) Predict the substrate range by applying the predictive model developed at steps 3a-3f, using interaction energies (from step 4b) as a sole input.

### Example set 2 - In silico screening of enzyme mutants with altered substrate specificity

### Context

Screen mutants of an enzyme *in silico* and determine their substrate range/preference. This can be used in the context of *in silico* enzyme design, to obtain a mutant with altered substrate specificity, for instance to enhance specificity (i.e. minimize the catalytic efficiency of the enzyme on undesired substrates), and/or to widen the substrate range of an enzyme and access substrates (hence, products) that are not (sufficiently) processed by the wild-type enzyme. This can be applied iteratively, i.e. several rounds of *in silico* mutations-screening can be performed to obtain the desired properties. The following examples focus on a single mutant enzyme, but can be extended to multiple mutant enzymes.

### Example 2a (basic)

1. Retrieve primary sequences of enzymes for which the substrate spectrum has been previously characterized: this includes the wild-type enzyme of interest and/or related enzymes. Also retrieve the corresponding substrate spectra. Optionally extended by experimentally determining the substrate spectrum of yet uncharacterized enzymes.
2. Process 'known' enzyme sequences (collected at step 1) as follows:
   a) to b) as in example 1a
   c) as in example 1a, except that additional ligands can be included (for instance, the ligand of interest, for which the mutant enzyme should display higher specificity and/or higher affinity/catalytic efficiency, provided experimental data is available regarding its conversion into product).
   d) to g) as in example 1a
3. Create a predictive model based on 'known' enzymes (collected at step 1):
   a) Divide the primary sequences (collected at step 1) into a training dataset and a validation dataset.
   b) Use any machine-learning model to develop and calibrate a model that ranks possible ligands according to their probability of being used as substrates by each of the known enzymes in the training dataset, based on their associated vectors of information (from step 2g) and known substrate spectrum (collected at step 1). The idea would be to initially use a simple model based on a linear combination of the terms in the vectors of information (typically, at least the interaction energy), possibly after transformation (log, ...) - however, this idea shifted based on the preference for version c (full) as described below.
   c) Validate the model developed at step 3b with the validation dataset, using only their associated vectors of information (from step 2g) as an input, and comparing the predicted ranked list of ligands with the experimentally determined ligands.
   d) Optionally, repeat steps 3a-3c iteratively with different machine-learning models and/or different training/validation datasets and/or different initial parameter values, and compare the predictions of the different models to select the best model.
**4. Determine the substrate range of the original and mutant enzymes:**
   a) Retrieve the primary sequences of interest.
   b) Derive a vector of information for the original and mutant enzymes by applying steps 2a-2g. Step 2c should include the ligand of interest, for which the mutant enzyme should display higher specificity and/or higher affinity/catalytic efficiency.
   c) Predict the substrate range of both enzymes by applying the predictive model developed at steps 3a-3d, using the vectors of information (from step 4b) as sole inputs.
   d) **Compare** the predicted substrate range of the mutant enzyme (including the ligand of interest, for which the mutant enzyme should display higher specificity and/or higher affinity/catalytic efficiency) to the predicted substrate range of the original enzyme, in order to decide whether the mutant enzyme displays optimized properties compared to the original enzyme.

### Example 2b (intermediate)

1. Retrieve primary sequences of enzymes for which the substrate spectrum has been previously characterized: this includes the wild-type enzyme of interest and/or related enzymes. Also retrieve the corresponding substrate spectra. Optionally extended by experimentally determining the substrate spectrum of yet uncharacterized enzymes.
2. Process 'known' enzyme sequences (collected at step 1) as follows:
   a) to f) as in example 2a
   g) to h) as in example 1b
3. Create a predictive model based on 'known' enzymes (collected at step 1):
   a) Divide the primary sequences (collected at step 1) into a training dataset and a validation dataset.
   b) Use any machine-learning model to develop and calibrate a model that ranks possible ligands according to their probability of being used as substrates by each of the known enzymes in the training dataset, based on their associated vectors of information (from step 2h) and known substrate spectrum (collected at step 1). The idea would be to initially use a simple model based on a linear combination of the terms in the vectors of information (typically, at least the interaction energy), possibly after transformation (log, ...) - however, this idea shifted based on the preference for version c (full) as described below.
   c) Validate the model developed at step 3b with the validation dataset, using only their associated vectors of information (from step 2h) as an input, and comparing the predicted ranked list of ligands with the experimentally determined ligands.
   d) Optionally, repeat steps 3a-3c iteratively with different machine-learning models and/or different training/validation datasets and/or different initial parameter values, and compare the predictions of the different models to select the best model.
**4. Determine the substrate range of the original and mutant enzymes:**
   a) Retrieve the primary sequences of interest.
   b) Derive a vector of information for the original and mutant enzymes by applying steps 2a-2h. Step 2c should include the ligand of interest, for which the mutant enzyme should display higher specificity and/or higher affinity/catalytic efficiency.
   c) Predict the substrate range of both enzymes by applying the predictive model developed at steps 3a-3d, using the vectors of information (from step 4b) as sole inputs.
   d) **Compare** the predicted substrate range of the mutant enzyme (including the ligand of interest, for which the mutant enzyme should display higher specificity and/or higher affinity/catalytic efficiency) to the predicted substrate range of the original enzyme, in order to decide whether the mutant enzyme displays optimized properties compared to the original enzyme.

### Example 2c (full)

1. Retrieve primary sequences of enzymes for which the substrate spectrum has been previously characterized: this includes the wild-type enzyme of interest and/or related enzymes. Also retrieve the corresponding substrate spectra. Optionally extended by experimentally determining the substrate spectrum of yet uncharacterized enzymes.
2. Process 'known' enzyme sequences (collected at step 1) as follows:
   a) to f) as in example 2a
   g) to h) as in example 1c
3. Create a predictive model based on 'known' enzymes (collected at step 1):
   a) Divide the primary sequences (collected at step 1) into a training dataset and a validation dataset.
   b) Develop a kinetic mechanistic model that uses the 3 interaction energies of the *izjs* poses (from step 2h) to simulate the time-dependent conversion of substrates to products. At minimum, the model is based on a biochemical reaction network describing, for each *izjs* pose, (i) binding of the substrate to the enzyme, (ii) conversion of the substrate into the reaction intermediate, and (iii) conversion of the reaction intermediate to the product. Reaction rates and equilibrium constants in the model are based on the difference in interaction energies between the different molecular species (substrate, reaction intermediate, product). The network can optionally include exchange reactions between different poses and/or conformers of the same ligand, at the substrate, reaction intermediate, or product stage; at the reaction intermediate stage, exchange reactions should be further restricted to exchange between identical stereoisomers.
   c) Develop a simulation framework to simulate the behavior of the kinetic model (from step 3b). More specifically, this framework should allow comparing all ligands for any given enzyme, in terms of their overall rate of transformation into their cognate product (i.e. considering all conformers and poses (*js*) for every substrate/stereoisomer (iz) pair). Ideally - but not necessarily - the simulation framework should take into account possible differences in concentrations between different substrates. The simulation framework according to the present method comprises performing stochastic simulations - either with all substrates together or with overlapping subsets of at least 2 different substrates - in order to determine a quantitative ranking of substrates for every enzyme.
   d) Calibrate the model parameters of step 3b with the training dataset, using the simulation framework of step 3c. This step consists in optimizing parameter values so that the input data (interaction energies for the substrate-enzyme, reaction intermediate-enzyme, and product-enzyme complexes; from step 2h) result in substrate ranking predictions as close as possible to the known substrate spectrum of the enzymes in the training dataset. For this, develop an objective function that combines the predicted vs. experimental relative substrate preference (qualitative ranking) and predicted vs. experimental absolute substrate preference (quantitative probability of selecting each substrate). Any minimization algorithm can be used to minimize this objective function by altering the parameter values in the kinetic model.
   e) Validate the model calibrated at step 3d with the validation dataset, using only their interaction energies for the substrate-enzyme, reaction intermediate-enzyme, and product-enzyme complexes (from step 2h) as an input, and comparing the predicted ranked list of substrates with the experimentally determined substrates. This will typically be performed using the same objective function as in step 3d.
   f) Optionally, repeat steps 3a-3e iteratively with different models (step 3b) and/or different training/validation datasets (steps 3a and 3e) and/or different simulation frameworks (step 3c) and/or different initial parameter values (step 3d) and/or different objective functions (step 3d), and compare the predictions of the different models to select the best model. Preferably, test variations of (i) the kinetic model (for instance, with or without inter-conformer exchanges), (ii) the simulation framework (for instance, simulations with all possible substrates at the same time vs. successive, overlapping 2-by-2 competitions), and (iii) the objective function (from fully quantitative ranking to semi-quantitative ranking).
   g) Optionally, steps 3a-3f can be modified to include direct competitions between enzymes. For instance, for the specific application of comparing different enzymes (original vs. mutant(s)) for a single substrate, the simulation framework could be transposed to competition(s) between the different enzyme(s), with the substrate of interest. The output of simulations would then be a quantitative ranking of the ability of the different enzymes to convert the substrate of interest into its product.
**4. Determine the substrate range of the original and mutant enzymes:**
   a) Retrieve the primary sequences of interest.
   b) Derive interaction energies for the substrate-enzyme, reaction intermediate-enzyme, and product-enzyme complexes, for the original and mutant enzymes, by applying steps 2a-2h. Step 2c should include the ligand of interest, for which the mutant enzyme should display higher specificity and/or higher affinity/catalytic efficiency.
   c) Predict the substrate range of both enzymes by applying the predictive model developed at steps 3a-3f, using interaction energies (from step 4b) as sole input.
   d) Compare the predicted substrate range of the mutant enzyme (including the ligand of interest, for which the mutant enzyme should display higher specificity and/or higher affinity/catalytic efficiency) to the predicted substrate range of the original enzyme, in order to decide whether the mutant enzyme displays optimized properties compared to the original enzyme.
      Note that if the simulation framework includes direct competition between the original and mutant enzyme for the ligand of interest, the comparison is straightforward and does not require predicting the full substrate spectrum of each enzyme.

### Example set 3 - Phenotype prediction from enzyme variants

### Context

Prediction of the impact of an unknown (uncharacterized) enzyme variant on a biological process (phenotype). The uncharacterized enzyme comes from a family of enzymes where the link between an enzyme variant and a particular phenotype is known. Examples of phenotypes of interest include, but are not limited to, the amount of fluorescence emitted by a fluorescent protein, the antibiotic activity of a compound, the growth of an organism, or, more generally, the presence or absence of a pathology.

### Example 3a (basic)

1. Retrieve primary sequences of variant enzymes for which the enzyme substrate spectrum has been previously characterized (known enzymes) also retrieve their associated phenotypes.
2. Process 'known' enzyme sequences (collected at step 1) as follows:
   a) to g) as in example 1a
3. Create a predictive model based on 'known' enzymes (collected at step 1):
   a) Divide the primary sequences (collected at step 1) into a training dataset and a validation dataset.
   b) Use a two-step model that first takes as input a vector of information (from step 2g) and output a vector where each position corresponds to the probability of a specific ligand being used as a substrate by the corresponding enzyme. The second part of this model takes as input the vector containing the probability of a ligand being used as a substrate and outputs the probability to observe a particular phenotype. The two models are optimized simultaneously during the training process to obtain phenotype predictions as close as possible to the known associate phenotype of the enzyme variants in the training dataset. Any minimization algorithm can be used to minimize this objective function by finding the best parameter values of the full model.
   c) Validate the model developed in step 3b with the validation dataset, using only their associated vectors of information (from step 2g) as an input, and comparing the predicted phenotype with the known phenotype associate to the enzyme variant.
   d) Optionally, repeat steps 3a-3c iteratively with different machine-learning models and/or different training/validation datasets and/or different initial parameter values, and compare the predictions of the different models to select the best model.
**4. Determine the substrate range of the unknown enzyme:**
   a) Retrieve the primary sequence of interest.
   b) Derive a vector of information for the unknown enzyme by applying steps 2a-2g.
   c) **Predict the phenotype of each enzyme variant** by applying the predictive model developed at steps 3a-3d, using the vector of information (from step 4b) as a sole input.
   d) **Obtain the substrate range prediction** by extracting the estimated probability of a ligand being used as substrate from the model at step 3b.

### Example 3b (intermediate)

1. Retrieve primary sequences of variant enzymes for which the enzyme substrate spectrum has been previously characterized (known enzymes) also retrieve their associated phenotypes.
2. Process 'known' enzyme sequences (collected at step 1) as follows:
   a) to f) as in example 1a
   g) to h) as in example 1b
3. Create a predictive model based on 'known' enzymes (collected at step 1):
   a) Divide the primary sequences (collected at step 1) into a training dataset and a validation dataset.
   b) Use a two-step model that first takes as input a vector of information (from step 2h) and output a vector where each position corresponds to the probability of a specific ligand being used as a substrate by the corresponding enzyme. The second part of this model takes as input the vector containing the probability of a ligand being used as a substrate and outputs the probability to observe a particular phenotype. The two models are optimized simultaneously during the training process to obtain phenotype predictions as close as possible to the known associate phenotype of the enzyme variants in the training dataset. Any minimization algorithm can be used to minimize this objective function by finding the best parameter values of the 2 models.
   c) Validate the model developed in step 3b with the validation dataset, using only their associated vectors of information (from step 2h) as an input, and comparing the predicted phenotype with the known phenotype associate to the enzyme variant.
   d) Optionally, repeat steps 3a-3c iteratively with different machine-learning models and/or different training/validation datasets and/or different initial parameter values, and compare the predictions of the different models to select the best model.
**4. Determine the substrate range of the unknown enzyme:**
   a) Retrieve the primary sequence of interest.
   b) Derive a vector of information for the unknown enzyme by applying steps 2a-2h.
   c) Predict the phenotype of each enzyme variant by applying the predictive model developed at steps 3a-3d, using the vector of information (from step 4b) as a sole input.
   d) Obtain the substrate range prediction by extracting the estimated probability of a ligand being used as substrate from the model at step 3b.

### Example 3c (full)

1. Retrieve primary sequences of variant enzymes for which the enzyme substrate spectrum has been previously characterized (known enzymes) also retrieve their associated phenotypes.
2. Process 'known' enzyme sequences (collected at step 1) as follows:
   a) to f) as in example 1a
   g) as in example 1c
3. Create a predictive model based on 'known' enzymes (collected at step 1):
   a) to c) as in example 1c
   d) Use a two-step model composed of kinetic mechanistic model of step 3b simulates by step 3c and a linear regression model. The linear regression model takes as input the vector containing the substrate ranking output by the simulated kinetic mechanistic model and outputs the probability to observe a particular phenotype. Calibrate the full model with the training dataset. This step consists in optimizing parameter values of the two steps model so that the input data (interaction energies for the substrate-enzyme, reaction intermediate-enzyme, and product-enzyme complexes; from step 2h) result in obtaining phenotype predictions as close as possible to the known associate phenotype of the enzyme variants in the training dataset. Any minimization algorithm can be used to minimize this objective function by finding the best parameter values of the model.
   e) Validate the model developed at step 3d with the validation dataset, using only their interaction energies for the substrate-enzyme, reaction intermediate-enzyme, and product-enzyme complexes (from step 2h) as an input, and comparing the predicted phenotype with the known phenotype associate to the enzyme variant.
   f) Optionally, repeat steps 3a-3e iteratively with different models (step 3b) and/or different training/validation datasets (steps 3a and 3e) and/or different simulation frameworks (step 3c) and/or different initial parameter values (step 3d) and/or different objective functions (step 3d), and compare the predictions of the different models to select the best model. Preferably test variations of (i) the kinetic model (for instance, with or without inter-conformer exchanges), (ii) the simulation framework (for instance, simulations with all possible substrates at the same time vs. successive, overlapping 2-by-2 competitions).
**4. Determine the substrate range of the unknown enzyme:**
   a) Retrieve the primary sequence of interest.
   b) Derive interaction energies for the substrate-enzyme, reaction intermediate-enzyme, and product-enzyme complexes, for the unknown enzyme, by applying steps 2a-2h.
   c) Predict the phenotype of each enzyme variant by applying the predictive model developed at steps 3a-3f, using interaction energies (from step 4b) as a sole input.
   d) Obtain the substrate range prediction by extracting the estimated probability of a ligand being used as substrate from the model at step 3d.

### Example 4: practical implementation for multi-step reactions based on the CDPS's reaction

As shown in figures 1 and 2, the CDPS-reaction is a multistep reaction involving three steps and using two substrates at steps 2-3 of the reaction. To illustrate how the steps of the computer-implemented method of the present invention are applied to each reaction of the CDPS reaction, we hereby consider an example for which a machine-learning model was trained beforehand. Reference is made to steps a) to g) of the computer-implemented method.

Each preceding reaction of a multi-step reaction acts as a means to reduce the set of potential substrates for the subsequent reaction by eliminating 'poor' substrates with low rankings given by the machine learning model. Having more than one substrate for the next reaction is possible.

The substrates set for each of the three reactions of the CDPS are:
- Reaction 1 substrates set = (set i = 20 amino acid-tRNA)
- Reaction 2 substrates set = (set i' = 20 amino acid-tRNA) + (subset of Reaction 1 substrates set)
- Reaction 3 substrates set = (empty) + (subset Reaction 2 substrates set)

In the first reaction, we generate the 3D structures for each j conformations of one or more enzymes of the same family (CDPs family) at step a). Then, at step b), we generate the 3D representation of reaction intermediates of each of i possible substrates from the set of substrates for the first reaction. In the case of the first CDPS reaction, this set of substrates corresponds to all possible amino acid-tRNA combinations, totaling 20 aa-tRNA. Steps c)-e) involve creating a vector of information for each docking pose of the enzyme with the substrate, the enzyme with the reaction intermediate, and the enzyme with the product of the substrate, as well as their interaction energies. We retrieve the trained machine-learning model of that reaction (step f). Then at step g), we apply the trained machine learning model to the dataset of the vector of information and interaction energies. The model assigns each substrate i a likelihood of conversion to its corresponding product. The product of this first reaction is the amino acid, free from its tRNA, and linked to the catalytic S37 residue of the enzyme, as shown in Figure 2 (top). In this case, the model will output 20 probabilities, one for each substrate or corresponding product. Then, for the second reaction, we retrieve the 3D structures for each j conformations of one or more enzymes of the same family computed at step a) of the first reaction. Then, at step b) we generate the 3D representation of reaction intermediates by selecting two substrates one from the set i' and one from the set i. Substrate from set i is selected based on the best-ranking substrates of the previous reaction (the X substrate with the highest probability or substrates with a probability over a certain threshold, let's say we have X=5 for this example). The second substrate is selected from the set i', corresponding to the 20 amino acid-tRNA. Once selected, the reaction intermediates are generated by linking one of the 5 selected substrates' corresponding product from set i to the S37 of the enzyme (we recreate the product of the previous reaction) and by linking to the selected aa-tRNA from set i' as shown in Figure 2 (middle). The steps c)-e) are applied to generate a vector of information for each docking pose of the enzyme with the substrate, the enzyme with the reaction intermediate, and the enzyme with the product of the substrate. We retrieve the trained machine-learning model of that reaction (step f). Then, at step g), we applied the trained machine learning model to the dataset of the vector of information. The product of the second reaction is the amino acid from set i linked to the S37 of the enzyme plus the amino acid of set i' linked to first amino acid free from its t-RNA and forming a dipeptide. The model returns, for each substrate combination, a probability of conversion to its product. In this case, the model will output 100 probabilities ((20 from set i')*(5 substrates i)).

For the third reaction, we retrieve the 3D structures for each j conformations of one or more enzymes of the same family computed at step a) of the first reaction. Then, at step b) we generate the 3D representation of reaction intermediates by selecting two substrates one from the set i' and one from the set i and generate the reaction intermediate at step b) by selecting the best ranking substrate combination of the previous reaction, (an amino acid substrate i and a second amino acid substrate i'). Let's say we select the 10 best products from the 100 substrates combination of the previous reaction as substrates to generate the reaction intermediate. Unlike the two first reactions, the set of substrates for this reaction doesn't add new molecules and only considers substrates combination of the last reaction. The steps c-e are applied to generate a vector of information for each docking pose of the enzyme with the substrate, the enzyme with the reaction intermediate, and the enzyme with the product of the substrate. We retrieve the trained machine-learning model of that reaction (step f). Then, at step g), we applied the trained machine learning model to the dataset of the vector of information. The model gives each substrate a probability of conversion to its product. The product of the final reaction is the cyclic dipeptide free from the enzyme. The model returns, for each substrate combination, a probability of conversion to its product. In this case, the model will output 10 probabilities (10 amongst the (20 from set i')*(5 substrates i)). Those 10 probabilities are then used to determine the products of the enzyme.

The present invention is in no way limited to the embodiments described in the examples and/or shown in the figures. On the contrary, methods according to the present invention may be realized in many different ways without departing from the scope of the invention.

## Claims

1. A computer-implemented method for ranking and/or identifying substrate-enzyme combinations according to the likelihood that a substrate undergoes catalytic conversion by an enzyme or inhibits the enzyme, the method comprising the following steps:
a) obtaining a 3D structure for each j conformations of one or more enzymes of a same family,
b) obtaining a 3D representation of reaction intermediates of each of i possible substrates of said j conformations in every possible stereochemical configuration z of the reaction intermediate,
c) molecular docking said 3D representations of the reaction intermediates of step b) against each 3D structure of said j conformations of step a), thereby generating a plurality of s docking poses of the substrate in the active site for each izj combination;
d) for each izjs docking pose obtained in step c), deriving a vector of information that represents the docking pose for each of the enzyme with the substrate, the enzyme with the reaction intermediate, and the enzyme with the product of the substrate;
e) optionally, where in step d) the vector of information does not comprise interaction energies, converting the vector of information to interaction energies for each of the enzyme with the substrate, the enzyme with the reaction intermediate, and the enzyme with the product of the substrate;
f) training a machine learning model:
- using a one-step approach, on a training dataset comprising a subset of the i possible substrates and a first subset of the one or more enzymes and the associated likelihoods of each substrate of the subset of substrates undergoing catalytic conversion by the enzymes of the first subset or inhibiting the enzymes of the first subset based on vectors of information as in step d) or their determined interaction energies as in step e), thereby obtaining a machine learning model trained to provide a ranking and/or to identify substrate-enzyme combinations according to the likelihood that a substrate undergoes catalytic conversion by an enzyme or inhibits the enzyme based on interaction energies or vectors of information of each of the enzyme with the substrate, the enzyme with the reaction intermediate, and the enzyme with the product of the substrate,
or
- using a two-step approach comprising a first and a second step, the first step comprising training the machine learning model on a training dataset comprising vectors of information as in step d) or interaction energies as in step e), and their associated likelihoods of each substrate undergoing catalytic conversion by the enzymes or inhibiting the enzymes based on their determined interaction energies or vectors of information, and the second step comprising training the machine learning model on a training dataset comprising likelihoods of each substrate undergoing catalytic conversion by the enzymes or inhibiting the enzymes based on their determined interaction energies or vectors of information, and their associated likelihoods of the enzymes providing a certain biological phenotype,
thereby obtaining a machine learning model trained: to provide a ranking and/or to identify substrate-enzyme combinations according to the likelihood that a substrate undergoes catalytic conversion by an enzyme or inhibits the enzyme based on interaction energies or vectors of information of each of the enzyme with the substrate, the enzyme with the reaction intermediate, and the enzyme with the product of the substrate, and
to provide a likelihood of an enzyme providing a certain biological phenotype according to the likelihood that a substrate undergoes catalytic conversion by an enzyme or inhibits the enzyme based on the vector of information or on interaction energies of each of the enzyme with the substrate, the enzyme with the reaction intermediate, and the enzyme with the product of the substrate; and
g) based on vectors of information and/or interaction energies determined in step d) or e), determining the likelihood that the substrate undergoes catalytic conversion by the enzyme or inhibits the enzyme, using the trained model of step f), and subsequently providing a ranking and/or identifying substrate-enzyme combinations based on said likelihood.

2. The computer-implemented method according to claim 1, wherein the 3D structures of the j conformations in step a) include a reference 3D structure obtained from a reference primary amino acid or nucleotide sequence for the enzyme and 3D structures of alternative conformations of the enzyme.

3. The computer-implemented method according to claim 1 or 2, wherein the substrate and/or enzyme are composed of residues, such as amino acid, nucleotide, or fatty acid residues, and wherein side chains of selected residues are allowed to be flexible during the molecular docking of step c), optionally wherein said flexible residues are identified by structural alignment against one or more reference sequences.

4. The computer-implemented method according to any one of the previous claims 1-3, wherein docking poses obtained in step c) are:
- filtered based on enzyme knowledge, optionally wherein filtering is based on recognition of atom bonds and an empirically determined threshold for the distance between key atoms, and/or
- refined by validation against an alternative docking algorithm, optionally wherein local re-optimization of poses is performed,
before vectors of information or interaction energies are determined.

5. The computer-implemented method according to any one of the previous claims 1-4, wherein the one or more enzymes of a same family comprises at least 1 enzyme of the same family.

6. The computer-implemented method according to any one of the previous claims 1-5, wherein enzymes of the same family exhibit similar substrate specificity, sharing the ability to catalyze reactions involving a comparable range of substrates, preferably as determined in silico or experimentally determined.

7. The computer-implemented method according to any one of the previous claims 1-6, wherein the method further comprises validating the machine learning model of step f):
▪ for the one-step approach, using a validation data set comprising the subset of i possible substrates and a second subset of the one or more enzymes, by providing the interaction energies and/or vector of information associated with the combinations of substrates and the enzymes in said validation dataset, and comparing the predicted ranked list of substrates with experimentally determined substrates, or
▪ for the two-step approach, using a validation data set comprising vectors of information as in step d) or interaction energies as in step e), and comparing predicted biological phenotypes with known or experimentally determined phenotypes associated with the enzyme.

8. A computer system for ranking and/or identifying substrate-enzyme combinations according to the likelihood that a substrate undergoes catalytic conversion by an enzyme or inhibits the enzyme, the computer system configured for performing the computer-implemented method of any one of the previous claims 1-7.

9. A computer program product for ranking and/or identifying substrate-enzyme combinations according to the likelihood that a substrate undergoes catalytic conversion by an enzyme or inhibits the enzyme, the computer program product comprising instructions which, when the computer program product is executed by a computer, cause the computer to carry out the computer-implemented method according to any one of preceding claims 1 to 7.

10. Use of the computer-implemented method according to any of preceding claims 1 to 7 and/or the computer system according to preceding claim 8 and/or the computer program product according to preceding claim 9, for predicting the substrate range of an enzyme X.

11. A computer-implemented method of predicting the substrate range of an enzyme X using the method of any one of the previous claims 1-7, comprising the steps of:
i) for said enzyme X, obtaining the vectors of information or interaction energies of each of enzyme X with the substrate, the enzyme X with the reaction intermediate, and the enzyme X with the product of the substrate, for each izjs pose of enzyme X as in steps a) to d) or a) to e) of claim 1, and
ii) using said vectors of information or interaction energies obtained in i), to predict the substrate range of enzyme X using the machine learning model of step f) of claim 1.

12. Use of the computer-implemented method according to any of preceding claims 1 to 7 and/or the computer system according to preceding claim 8 and/or the computer program product according to preceding claim 9, for screening enzyme variants with altered substrate specificity and/or catalytic activity in relation to a substrate of interest.

13. A computer-implemented method of screening enzyme variants with altered substrate specificity and/or catalytic activity in relation to a substrate of interest, using the method of any one of the previous claims 1-7, comprising the steps of:
i) for a plurality of said enzyme variants and a reference enzyme, obtaining the vectors of information or interaction energies of each of the enzymes with the substrate, the enzymes with the reaction intermediate, and the enzymes with the product of the substrate, for each izjs pose as in steps a) to d) or a) to e) of claim 1, and
ii) using said vector of information or interaction energies obtained in i), identifying an enzyme variant having an altered substrate specificity and/or catalytic activity compared to the reference enzyme using the machine learning model of step f) of claim 1.

14. The computer-implemented method according to claim 13, wherein step ii) comprises:
▪ predicting the substrate range of the reference enzyme and of the enzyme variants based on the vectors of information or interaction energies in step i) using the machine learning model of step f) of claim 1,
▪ comparing the predicted substrate ranges of the reference enzyme with the predicted substrate ranges of the enzyme variants, and
▪ identifying an enzyme variant having an altered substrate specificity and/or catalytic activity compared to the reference enzyme.

15. The computer-implemented method according to claim 13 or 14, wherein the substrate range includes the substrate of interest.

16. The computer-implemented method according to conclusion 13 to 15, wherein said altered substrate specificity in relation to a substrate of interest includes a higher specificity, a higher affinity, and/or a higher catalytic efficiency of the enzyme variant compared to the reference enzyme.

17. The computer-implemented method according to any one of the previous claims 13 to 16, wherein the steps are repeated to screen a plurality of substrates.

18. Use of the computer-implemented method according to any of preceding claims 1 to 7 and/or the computer system according to preceding claim 8 and/or the computer program product according to preceding claim 9, wherein the machine learning model is trained using the two-step approach, for predicting the biological phenotype associated with an enzyme X.

19. A computer-implemented method of predicting the biological phenotype associated with an enzyme X using the method of any one of the previous claims 1-7, comprising the steps of:
i) for said enzyme X, obtaining the vectors of information or interaction energies of each of enzyme X with the substrate, the enzyme X with the reaction intermediate, and the enzyme X with the product of the substrate, for each izjs pose of enzyme X as in steps a) to d) or a) to e) of claim 1, and
ii) using said vectors of information or interaction energies obtained in i), to predict biological phenotype associated with enzyme X using the machine learning model of step f) of claim 1 using the two-step approach.
